# EUROPEAN PATENT SPECIFICATION

(11) **EP 4 065 055 B1**
(45) Date of publication and mention of the grant of the patent: **03.04.2024**
(21) Application number: 21704973.3
(22) Date of filing: 08.01.2021
(51) Int. Cl.: A61M 25/06, A61F 2/24, A61M 25/00, A61F 2/95

(54) **INTRODUCER SHEATH WITH CAMMING TIP**
EINFÜHRERHÜLSE MIT NOCKENSPITZE
GAINE D'INTRODUCTION AVEC POINTE DE CAME

(30) Priority: 16.01.2020 US 202062961913 P
(43) Date of publication of application: 05.10.2022
(60) Divisional application: 23209684.2
(73) Proprietor: Edwards Lifesciences Corporation, Irvine, CA 92614-5688 (US)
(72) Inventor: CUMMINGS, Brendan, Christopher, Costa Mesa, CA 92626 (US)
(74) Representative: Venner, Julia Ann
(86) International application number: PCT/US2021/012670
(87) International publication number: WO 2021/146110

(56) References cited:
- FR-A1- 2 707 505
- US-A1- 2004 049 226
- US-A1- 2006 030 814
- US-A1- 2012 035 700
- US-B1- 6 565 536
- US-B2- 8 790 387

## Description

This application claims the benefit of U.S. Provisional Patent Application No. 62/961,913, filed on January 16, 2020, entitled INTRODUCER SHEATH WITH CAMMING TIP.

### FIELD

The present application concerns embodiments of a sheath for use with catheter-based technologies for repairing and/or replacing heart valves, as well as for delivering an implant, such as a prosthetic valve to a heart via the patient's vasculature.

### BACKGROUND

Endovascular delivery catheter assemblies are used to implant prosthetic devices, such as a prosthetic valve, at locations inside the body that are not readily accessible by surgery or where access without invasive surgery is desirable. For example, aortic, mitral, tricuspid, and/or pulmonary prosthetic valves can be delivered to a treatment site using minimally invasive surgical techniques.

An introducer sheath can be used to safely introduce a delivery apparatus into a patient's vasculature (e.g., the femoral artery). An introducer sheath generally has an elongated sleeve that is inserted into the vasculature and a housing that contains one or more sealing valves that allow a delivery apparatus to be placed in fluid communication with the vasculature with minimal blood loss. A conventional introducer sheath typically requires a tubular loader to be inserted through the seals in the housing to provide an unobstructed path through the housing for a valve mounted on a balloon catheter. A conventional loader extends from the proximal end of the introducer sheath, and therefore decreases the available working length of the delivery apparatus that can be inserted through the sheath and into the body.

Conventional methods of accessing a vessel, such as a femoral artery, prior to introducing the delivery system include dilating the vessel using multiple dilators or sheaths that progressively increase in diameter. This repeated insertion and vessel dilation can increase the amount of time the procedure takes, as well as the risk of damage to the vessel.

Radially expanding intravascular sheaths have been disclosed. Such sheaths tend to have complex mechanisms, such as ratcheting mechanisms that maintain the shaft or sheath in an expanded configuration once a device with a larger diameter than the sheath's original diameter is introduced.

However, delivery, removal and/or repositioning of prosthetic devices within a patient still poses a risk to the patient. Furthermore, accessing the vessel remains a challenge due to the relatively large profile of the delivery system that can cause longitudinal and radial tearing of the vessel during insertion. The delivery system can additionally dislodge calcified plaque within the vessels, posing an additional risk of clots caused by the dislodged plaque.

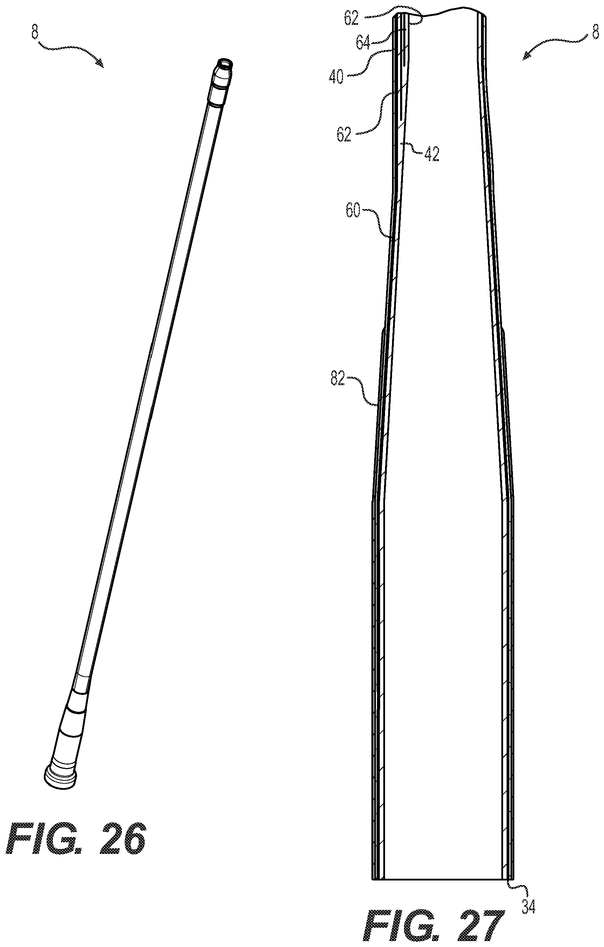

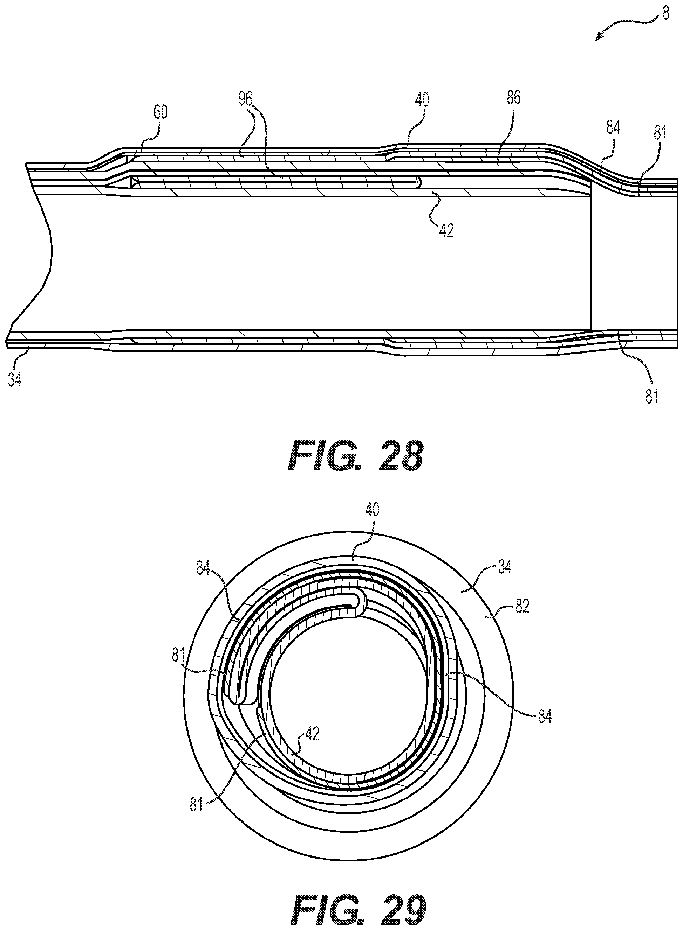

U.S. Patent No. 8,790,387, which is entitled "Expandable Sheath for Introducing An Endovascular Delivery Device Into A Body", discloses a sheath with a split outer polymeric tubular layer and an inner polymeric layer, for example in FIGS. 27A and 28. A portion of the inner polymeric layer extends through a gap created by the cut and can be compressed between the portions of the outer polymeric tubular layer. Upon expansion of the sheath, portions of the outer polymeric tubular layer have separated from one another, and the inner polymeric layer is expanded to a substantially cylindrical tube. Advantageously, the sheath disclosed in the '387 patent can temporarily expand for passage of implantable devices and then return to its starting diameter.

Despite the disclosure of the '387 patent, there remains a need for further improvements in introducer sheaths for endovascular systems used for implanting valves and other prosthetic devices.

US 2012/0035700 describes a drainage stent delivery system including an elongate shaft of a medical device, a drainage catheter or stent, and a retention mechanism for selectively retaining the drainage stent on the elongate shaft. The tubular stent is positioned on and surrounding the elongate shaft. The elongate shaft includes a distal tip portion which is deflectable from a first position to a second position, the distal tip portion of the elongate shaft being biased toward the first position. Deflecting the distal tip portion of the elongate shaft from the first position to the second position moves the distal tip portion toward the central longitudinal axis of the tubular stent to allow the stent to be decoupled from the elongate shaft.

US 2004/0049226 describes a retrieval catheter for retrieving a medical device deployed in a vasculature. The catheter comprises an outer catheter body and an inner coupling member having means for coupling to a medical device deployed in a vasculature. The catheter body is movable distally relative to the coupling member to retrieve a coupled medical device into the catheter body.

US 6,565,536 describes a catheter device for fixation to the human body which comprises a catheter having a distal end and a proximal end, the distal end comprising one or more fins, the fins having an open position defining a large diameter and a closed position of small diameter. The catheter has a catheter body and a push tube sliding axially within the catheter body for manipulating the fins into the open and closed positions.

### SUMMARY

An aspect of the presently claimed invention is set out in the independent claim. Particular embodiments of this aspect are set out in the dependent claims. Any subject matter contained herein that does not fall within the scope of the appended claims is considered as being useful for understanding the invention.

The needs above and other advantages are provided by an introducer sheath system. The introducer disclosed herein can be used to introduce a variety of medical devices. Multiple devices can be sequentially introduced into a body cavity through the same device introducer, thereby reducing the potential for trauma to the body tissue, and reducing the number of incisions. The introducers disclosed herein are particularly useful in minimally invasive procedures where surgical space is limited for multiple device insertion. It will be understood that the introducer can also be used on non-vascular tissues, e.g., the stomach during gastrostomy tube insertion, or on the bile duct during stent placement.

The introducer sheath system includes a sheath member defining a tubular structure extending between a proximal end and a distal end of the sheath. The system can include a tip portion provided at the distal end of the sheath member. The tip portion defines a tubular structure extending between a proximal end and a distal end of the tip portion. The tip portion has a central lumen extending therethrough. The tip portion includes a camming feature formed as a protrusion from the central lumen of the tip portion extending inwards towards the longitudinal axis of the sheath. The tip portion is movable between a non-expanded configuration when the camming feature is not engaged and an expanded configuration when the camming feature is engaged. When the tip portion is in the non-expanded configuration, a diameter of the distal end of the tip portion is less than a diameter of the proximal end of the tip portion.

In some embodiments, when the tip portion is in the non-expanded configuration, at least a portion of an outer surface of the tip portion defines a decreasing tapered surface. The decreasing tapered surface extends between the proximal and distal ends of the tip portion.

In some embodiments, the sheath member and the tip portion form a continuous central lumen. The continuous central lumen extends between the proximal end of the sheath member and the distal end of the tip portion.

In some embodiments, the camming feature is formed as a curvilinear surface projecting from the central lumen of the tip portion. The curvilinear surface includes a uniform convex surface.

In the presently claimed invention, the camming surface includes a leading edge and an adjoining trailing edge, the leading edge adj acent the proximal end of the tip portion and the trailing edge adjacent the distal end of the tip portion. In some embodiments, a slope of the leading edge is less than a slope of the trailing edge.

In some embodiments, a thickness of the tip portion along the camming feature is greater than a thickness along a portion of the tip portion excluding the camming feature.

In some embodiments, at least a portion of the tip portion deforms upon transition between the non-expanded and expanded configurations. This allows the distal end of the tip portion to flare open to a larger diameter in the expanded configuration.

In some implementations, a thickness of the camming feature remains constant in both the expanded and non-expanded configuration.

In some implementations, the tip portion is biased to the non-expanded configuration.

In some embodiments, when the tip portion is in the expanded configuration, a portion of an outer surface of the tip portion forms an increasing tapered surface.

In some embodiments, when the tip portion is in the expanded configuration, the inner diameter of the tip portion increases between a proximal end of the tip portion and a distal end of the tip portion, forming a funnel-like shape.

In some embodiments, the introducer sheath system includes a catheter, wherein the catheter is at least partially disposed within a central lumen of the sheath member and movable longitudinally therethrough. The catheter is movable through the central lumen of the tip portion. Engagement between the catheter and the camming surface causes the tip portion to transition from the non-expanded configuration to expanded configuration.

In some embodiments, the sheath member is radially expandable.

In some embodiments, the proximal end of the tip portion is formed from an elastomer material.

In some embodiments, the sheath member and the tip portion are formed from an elastomer material.

Also disclosed herein, but not covered by the presently-claimed invention, is a method of delivering a medical device. The method includes inserting an introducer sheath into a blood vessel. The introducer sheath includes a sheath member defining a tubular structure extending between a proximal end and a distal end of the sheath. The introducer sheath includes a tip portion provided at the distal end of the sheath member. The tip portion defines a tubular structure extending between a proximal end and a distal end of the tip portion. The tip portion has a central lumen extending therethrough. The tip portion includes a camming feature as a protrusion from the central lumen of the tip portion. The tip portion is movable between a non-expanded configuration when the camming feature is not engaged and an expanded configuration when the camming feature is engaged. When the tip portion is in the non-expanded configuration, a diameter of the distal end of the tip portion is less than a diameter of the proximal end of the tip portion.

The method also includes advancing a payload through the sheath member to the tip portion and engaging the camming feature with the payload to transition the tip portion from the non-expanded to the expanded configuration. The method includes extending at least part of the payload past the distal end of the tip portion while maintaining the tip portion in the expanded configuration. The method includes retracting at least part of the payload through the distal end of the tip portion in a direction toward the proximal end of the tip portion. The method includes disengaging the camming feature such that the tip portion transitions from the expanded to the non-expanded configuration.

The camming feature may be formed as a curvilinear surface projecting from the central lumen of the tip portion, the curvilinear surface having a leading edge and an adjoining trailing edge. The leading edge is adjacent the proximal end of the tip portion and the trailing edge adjacent the distal end of the tip portion. Engaging the camming feature includes applying a force to a leading edge of the camming surface. Disengaging the camming feature comprises removing the force from the leading edge of the camming surface.

The method may include completely removing the payload from the introducer sheath and the tip portion.

The payload may be a catheter body.

The method may include removing the sheath member from the venous structure.

### DESCRIPTION OF DRAWINGS

FIG. 1 is an elevation view of an expandable sheath along with an endovascular delivery apparatus for implanting a prosthetic implant.
FIG. 2 is a cross sectional view of a sheath and a hub.
FIG. 3A is a magnified view of distal tip of the sheath.
FIG. 3B is a cross sectional view of the distal tip of the sheath, taken along line 3B-3B of FIG. 3A.
FIG. 4 is a cross sectional view of an exemplary implementation of the outer tubular layer of the sheath.
FIG. 5 is a cross sectional view of another exemplary implementation of the outer tubular layer of the sheath.
FIG. 6 is a magnified view of part of the outer tubular layer of FIG. 5, showing the cross section of longitudinal rods in greater detail.
FIG. 7 is a cross section of an exemplary implementation of the inner tubular layer of the sheath.
FIG. 8 is a cross section of both the inner and outer tubular layers of the sheath. In this example, the inner tubular layer is in the compressed condition.
FIG. 9 is a perspective view of the distal end of an implementation of the expandable sheath.
FIG. 10 is a side view of one implementation of the expandable sheath.
FIG. 11 is a perspective view of one embodiment of a flared distal portion of the sheath.
FIG. 12 shows a side view of the distal portion of a sheath folded in a heat-shrink tube.
FIG. 13 shows a longitudinal cross section of an embodiment of the distal portion of the sheath including a radioopaque tubular layer.
FIG. 14 shows an example flared distal portion of a sheath in a folded configuration.
FIG. 15 shows a cross section of a distal portion of a sheath in a folded configuration.
FIG. 16 shows a sheath during passage of an implant. The inner and outer tubular layers are adhered together in a longitudinally extending strip.
FIG. 17 shows a cross section of an exemplary embodiment including longitudinal rods embedded in the outer tubular layer and protruding into the elastic lumen.
FIG. 18 shows a cross section of an exemplary embodiment including longitudinal rods embedded in the outer tubular layer and protruding into the elastic lumen and outward from the outer surface of the outer tubular layer.
FIG. 19 shows a cross section of an exemplary embodiment including longitudinal rods embedded in the outer tubular layer, where some rods protrude into the elastic lumen and others protrude outward from the outer surface of the outer tubular layer.
FIG. 20 shows a cross section of an exemplary embodiment including longitudinal rods embedded in the outer tubular layer and the inner tubular layer. The longitudinal rods embedded in the outer tubular layer protrude into the elastic lumen and the longitudinal rods embedded in the inner tubular layer protrude into the central lumen.
FIG. 21 shows a cross section of another exemplary embodiment including longitudinal rods embedded in the outer tubular layer and protruding into the elastic lumen.
FIG. 22 shows a side view of the sheath with an implant passing therethrough.
FIG. 23 shows a flared implementation of a distal portion of the sheath, where the flared portion is folded into a compressed configuration.
FIG. 24 shows the distal portion of FIG. 23 with the flared portion unfolded and expanded.
FIG. 25 shows a cross section of the distal portion of FIG. 23, where the flared portion is folded into a compressed condition.
FIG. 26 shows a perspective view of an exemplary implementation of the expandable sheath.
FIG. 27 shows a longitudinal cross section of the proximal region of the implementation shown in FIG. 26.
FIG. 28 shows a longitudinal cross section of the distal region of the implementation shown in FIG. 26.
FIG. 29 shows a cross section of the distal region of the implementation shown in FIG. 26.
FIGS. 30 through 38 show a method of assembling a stiffened and sealed tip for another embodiment of the expandable sheath.
FIG. 39 shows a perspective view of an expandable introducer sheath having an expandable tip portion.
FIG. 40A shows a partial side view of the tip portion of the sheath in a non-expanded configuration.
FIG. 40B shows a cross section view of the tip portion of FIG. 40A.
FIG. 40C provides an enlarged view of Area A of FIG. 40B.
FIG. 40D provides an enlarged view of Area A of FIG. 40B.
FIG. 41A shows a partial side perspective view of the tip portion in a non-expanded configuration.
FIG. 41B shows a cross section view of the tip portion of FIG. 40A.
FIG. 42A shows a partial side view of the tip portion in the expanded configuration.
FIG. 42B shows cross section view of the tip portion of FIG. 42A.
FIG. 43A shows a partial cross section view of an example payload advancing to the distal end of the sheath, where the tip portion is in a non-expanded configuration.
FIG. 43B shows a partial cross section view of an example payload advancing through the distal end of the sheath, where the tip portion is in an expanded configuration.
FIG. 43C shows a partial perspective view of the tip portion of the sheath in the expanded configuration.

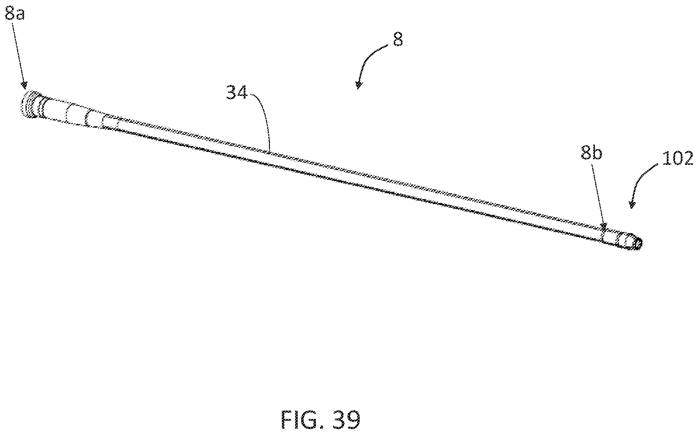

Figures 39 to 43 illustrate embodiments of the presently-claimed invention.

### DETAILED DESCRIPTION

Other advantages of the invention will become apparent to those skilled in the art from the following description.

For purposes of this description, certain aspects, advantages, and novel features of the embodiments and examples of this disclosure are described herein. The described methods, systems, and
apparatus should not be construed as limiting in any way. Instead, the present disclosure is directed toward all novel and nonobvious features and aspects of the various disclosed embodiments, alone and in various combinations and sub-combinations with one another. The disclosed methods, systems, and apparatus are not limited to any specific aspect, feature, or combination thereof, nor do the disclosed methods, systems, and apparatus require that any one or more specific advantages be present or problems be solved.

Features, integers, characteristics, compounds, chemical moieties, or groups described in conjunction with a particular aspect, embodiment or example of the invention are to be understood to be applicable to any other aspect, embodiment or example described herein unless incompatible therewith. All of the features disclosed in this specification (including any accompanying claims, abstract, and drawings), and/or all of the steps of any method or process so disclosed, may be combined in any combination, except combinations where at least some of such features and/or steps are mutually exclusive. The invention is not restricted to the details of any foregoing embodiments.

As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

"Optional" or "optionally" means that the subsequently described event or circumstance may or may not occur, and that the description includes instances where said event or circumstance occurs and instances where it does not.

Throughout the description and claims of this specification, the word "comprise" and variations of the word, such as "comprising" and "comprises," means "including but not limited to," and is not intended to exclude, for example, other additives, components, integers or steps. "Exemplary" means "an example of" and is not intended to convey an indication of a preferred or ideal aspect. "Such as" is not used in a restrictive sense, but for explanatory purposes.

Disclosed embodiments of an expandable sheath can minimize trauma to the vessel by allowing for temporary expansion of a portion of the introducer sheath to accommodate the delivery system, followed by a return to the original diameter once the device passes through. The expandable sheath can include, for example, an integrally formed inner tubular layer with thick and thin wall portions, wherein the thin wall portion can expand to an expanded lumen for passage of an implant and then fold back onto itself under biasing of an outer elastic tubular layer after departure of the implant. An expandable sheath is described in U.S. Patent Application No. 15/914,748 (published as U.S. Patent Application Publication No. 2018-0256858) entitled "Expandable Sheath with Longitudinally Extending Reinforcing Members,".

The expandable sheath can also include, for example, longitudinal cuts and/or break-away portions that, along with corresponding folded portions, facilitate expansion of the sheath during passage of an implant. An example of such expandable sheath is described in U.S. Patent No. 9,301,840 entitled "Expandable Introducer Sheath,".

In another aspect, the expandable sheath can include one or more longitudinally oriented stiffening elements (such as rods) that are coupled to the elastic outer layer to provide stiffness for the expandable sheath. Some embodiments can comprise a sheath with a smaller profile than the profiles of prior art introducer sheaths. Furthermore, present embodiments can reduce the length of time a procedure takes, as well as reduce the risk of a longitudinal or radial vessel tear, or plaque dislodgement because only one sheath is required, rather than several different sizes of sheaths. Embodiments of the present expandable sheath can avoid the need for multiple insertions for the dilation of the vessel.

Embodiments on the present introducer sheath can include tip structure that facilitates retrieval/withdraw of the implant back into the sheath while avoiding trauma to the vessel and damage to the implant and/or sheath. Retrieval/withdraw of the implant may be necessary during removal of the implant and/or repositioning of the implant within the vessel. For example, embodiments of the introducer sheath may include a distal tip that provides a tapered shape during insertion and flares during delivery/passage of the implant providing a funnel shape that facilitates withdraw of the implant back into the sheath.

Disclosed herein are elongate delivery sheaths and introducers that are particularly suitable for delivery of implants in the form of implantable heart valves, such as balloon-expandable implantable heart valves. Balloon-expandable implantable heart valves are well-known and will not be described in detail here. An example of such an implantable heart valve is described in U.S. Patent No. 5,411,552, and also in U.S. Patent Application Publication No. 2012/0123529. The elongate delivery sheaths disclosed herein may also be used to deliver other types of implantable devices, such as self-expanding implantable heart valves, stents or filters. The term "implantable" as used herein is broadly defined to mean anything - prosthetic or not - that is delivered to a site within a body. A diagnostic device, for example, may be an implantable.

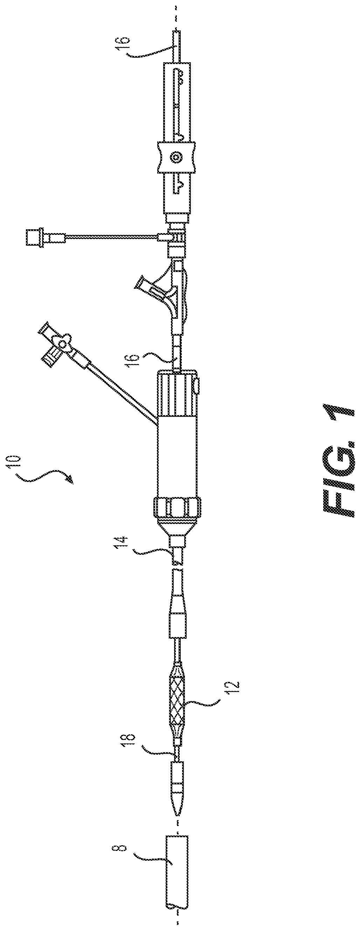

FIG. 1 illustrates an exemplary sheath 8 in use with a representative delivery apparatus 10, for delivering an implant 12, or other type of implantable, to a patient. The apparatus 10 can include a steerable guide catheter 14 (also referred to as a flex catheter) and a balloon catheter 16 extending through the guide catheter 14. The guide catheter 14 and the balloon catheter 16 in the illustrated embodiment are adapted to slide longitudinally relative to each other to facilitate delivery and positioning of the implant 12 at an implantation site in a patient's body, as described in detail below. The sheath 8 is an elongate, expandable tube that can include a hemostasis valve at the opposite, proximal end of the sheath to stop blood leakage.

Generally, during use a distal end of the sheath 8 is passed through the skin of the patient and inserted into a vessel, such as the trans-femoral vessel. The delivery apparatus 10 can be inserted into the sheath 8 through the hemostasis valve, and the implant 12 can then be delivered and implanted within the patient.

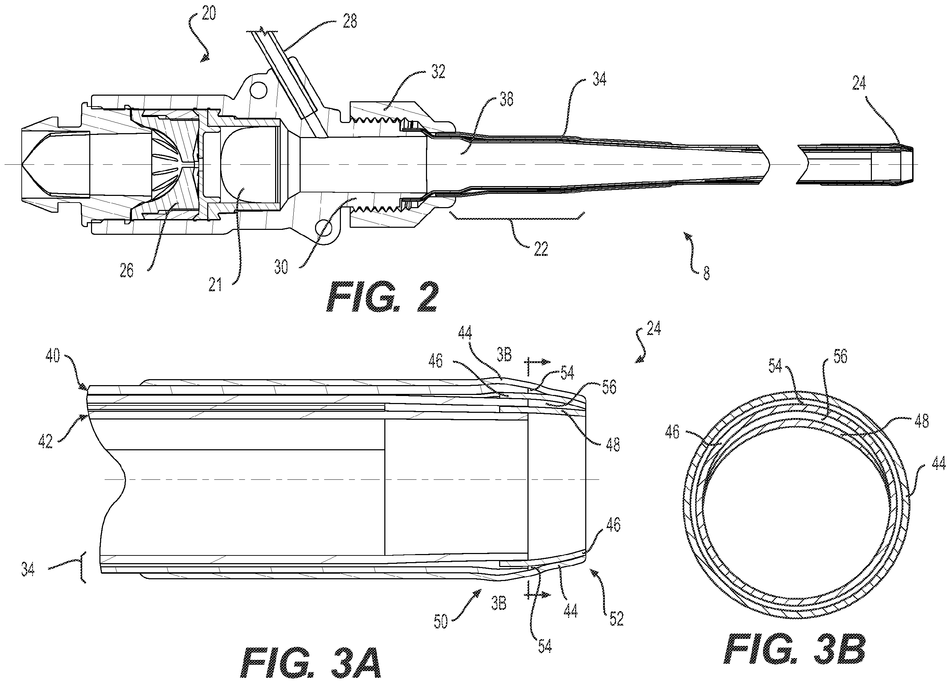

As shown in FIG. 2, the sheath 8 includes a hub 20, a flared proximal end 22 and a distal tip 24. The hub 20 is constructed of a rigid cylindrical structure defining a hub lumen 21 and houses a hemostasis valve 26 and may define a side port 28 and have a threaded distal end 30. The flared proximal end 22 of the sheath 8 includes a threaded female connector 32 mounted on a tubular wall structure 34. The distal tip 24 of the sheath 8 is mounted over a distal end of the tubular wall structure 34, as shown in FIG. 3. The tubular wall structure 34 defines a central lumen 38.

The hub 20 is attached to the flared proximal end 22 by twisting the threaded distal male end 30 into correspondingly threaded female connector 32. This places the hub lumen 21 in communication with the central lumen 38 of the tubular wall structure 34. The hemostasis valve 26 mediates access by the delivery apparatus 10 to the hub lumen 21 and central lumen 38 and ultimate deployment of the implant 12 in a pressurized (blood filled) environment. Side port 28 provides an additional access for application of saline or other fluids.

The distal tip 24, meanwhile, provides some restraint to the otherwise radially expandable tubular wall structure 34. The distal tip 24 also helps with advancement over an introducer by providing a tapered advancement surface. Further the distal tip 24 improves the stiffness of the sheath 8 at its distal tip to guard against buckling or collapse of the tubular wall structure 34 during torque and advancement forces.

As shown in FIG. 3A, the tubular wall structure 34 includes an elastic outer tubular layer 40 and an inner tubular layer 42 and the distal tip 24. The distal tip 24 generally has a tubular structure with a slightly tapering or frusto-conical distal end. The distal tip 24 includes an outer wall 44, an inner wall 46 and a retainer 48. The outer wall 44 has an axial length longer than the inner wall 46. A proximal end of the outer wall 44 has a tubular shape with straight sides. The outer wall tapers to a neck 52 at its distal free end and begins to flare slightly to a cylindrical bulge 50 moving proximally from the distal free end. The neck 52 has a smaller diameter than the proximal tubular end of the outer wall 44. The proximal tubular end in turn has a smaller diameter than the cylindrical bulge 50.

The inner wall 46 has a shorter axial length than the outer wall but also has a cylindrical shape that tapers - although more gradually - toward its distal free end. An outer surface of the inner wall 46 and inner surface of the outer wall 44 define an annular space 54 which is configured to receive a distal free end of the elastic outer tubular layer 40, as shown in FIG. 3A. The annular space 54 bulges some due to its position subjacent the cylindrical bulge 50 of the outer wall 44. This bulge facilitates insertion and capture of the elastic outer tubular layer. The annular space 54 tapers to a point moving distally as the surfaces of the outer wall 44 and inner wall 46 converge into binding contact.

The retainer 48 is an additional arc-shaped wall that extends along a portion of the inner surface of the inner wall 46 and defines its own crescent-shaped space 56, as shown in the cross section of FIG. 3B. The crescent-shaped space 56 is configured to receive a foldable thin wall portion of the inner tubular layer 42, as will be described in more detail below. The retainer 48 has an arc size that corresponds with a circumferential arc-length of the folded over portion of the inner tubular layer 42 when it is in its compressed or folded configuration. Advantageously, the distal tip 24 helps to increase the structural rigidity of the distal end of the tubular wall structure 34, blocks blood flow between the layers and provides a smooth, tapered profile for pushing through tissue when advanced over a wire or dilator.

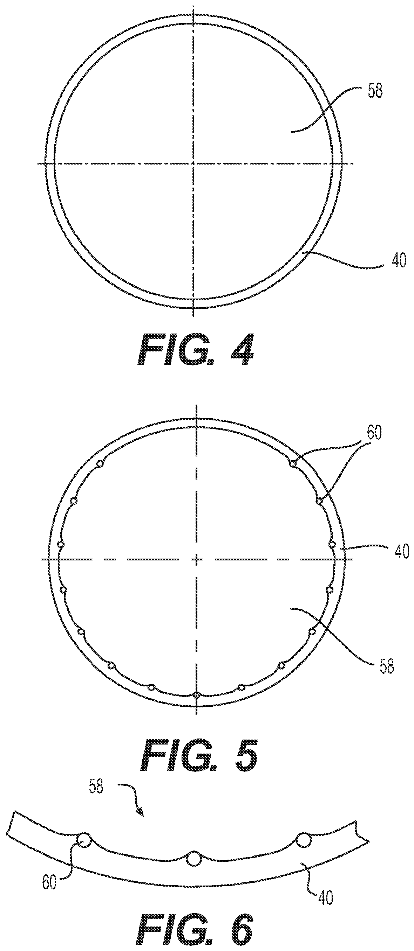

As shown in FIG. 4, the outer tubular layer 40 of one embodiment has a cylindrical shape with a circular cross-section along its entire length. The outer tubular layer 40 defines an initial elastic lumen 58 extending axially through its cylindrical cross-section. The outer tubular layer is sized to accommodate the delivery passage of the patient and/or the size of the implant 12 to be delivered. For example, the inside diameter, ID, of the layer 40 may be 0.4699 cm (0.185 inches) and may have a wall thickness of 0.0127+/- 0.00254 cm (0.005 +/- 0.001 inches) for delivery of a stent-mounted heart valve through trans-femoral access. In one aspect, inner surface of the outer tubular layer 40 and/or outer surface of the inner tubular layer 42 may be treated to have or have applied thereto a lubricious coating to facilitate unfolding and folding of the inner tubular layer 42.

The elastic lumen 58 is referred to as "initial" to designate its passive or as-formed diameter or cross-sectional dimension when not under the influence of outside forces, such as the implant 12 passing therethrough. It should be noted, however, that because the outer tubular layer 40 is comprised in the illustrated embodiment by an elastic material it may not retain its shape under even light forces such as gravity. Also, the outer tubular layer 40 need not have a cylindrical cross-section and instead could have oval, square or other cross-sections which generally can be configured to meet the requirements of the inner tubular layer 42 and/or expected shape of the implant 12. Thus, the term "tube" or "tubular" as used herein is not meant to limit shapes to circular cross-sections. Instead, tube or tubular can refer to any elongate structure with a closed-cross section and lumen extending axially therethrough. A tube may also have some selectively located slits or openings therein - although it still will provide enough of a closed structure to contain other components within its lumen(s).

The outer tubular layer 40, in one implementation, is constructed of a relatively elastic material that has enough flexibility to mediate the expansion induced by passage of the implant 12 and expansion of the inner tubular layer 42 while at the same time having enough material stiffness to urge the inner tubular layer back into an approximation of the initial diameter once the implant has passed. An exemplary material includes NEUSOFT. NEUSOFT is a translucent polyether urethane based material with good elasticity, vibration dampening, abrasion and tear resistance. The polyurethanes are chemically resistant to hydrolysis and suitable for overmolding on polyolefins, ABS, PC, Pebax and nylon. The polyuerthane provides a good moisture and oxygen barrier as well as UV stability. One advantage of the outer tubular layer 40 is that it provides a fluid barrier for the pressurized blood. Other materials having similar properties of elasticity may also be used for the elastic outer tubular layer 40.

FIG. 5 shows another implementation of the elastic outer tubular layer 40 including a plurality of longitudinal rods 60. The longitudinal rods 60 extend the length of the outer tubular layer 40 and protrude into the initial elastic lumen 58. The longitudinal rods 60 are coupled to the outer tubular layer, such as by being co-extruded and/or embedded into the elastic material of the outer tubular layer, as shown in FIG. 6. Advantageously, the longitudinal rods 60 are configured to provide a bearing surface to facilitate relative movement of the inner tubular layer 42 within the outer tubular layer 40. This is especially helpful when the inner tubular layer 42 is unfolding and returning to its originally folded shape.

The longitudinal rods 60 may be circumferentially spaced about the inside surface of the outer tubular layer 60. Although fifteen longitudinal rods 60 are shown in the cross-section of FIG. 5, any number, including a single one, of longitudinal rods may be employed. Also, the longitudinal rods 60 need not extend the entire length of the outer tubular layer 60. They may instead be applied selectively depending upon the demands of the implant, application and other circumstances. Longitudinal rods 60 may be selectively left out of an overall spacing pattern, such as in FIG. 5 where approximately 90 degrees of the inside surface of the outer tubular layer 40 is left as an unadorned surface.

As shown in FIG. 6, the longitudinal rods may have a circular cross-section so as to present a curved bearing surface into the elastic lumen 58. Although diameters for the longitudinal rods 60 may vary, in one embodiment they are 0.01016 cm (0.004 inches) in diameter. The outermost part of the longitudinal rod is positioned about 0.01524 cm (0.006 inches) from the outside surface of the outer tubular layer 40. In this manner, the inner edge surface of the longitudinal rods 60 spaces the inner tubular layer 42 from the surface of the outer tubular layer 40, thus reducing friction or the tendency to stick and impede relative movement. In other embodiments, the longitudinal rods can have other shapes, and the shapes may change within a single rod along the longitudinal direction. As also shown in FIG. 6, the material of the outer tubular layer 40 extends up in a slope past the midpoint of the cross-section of the longitudinal rods 60 for extra stability.

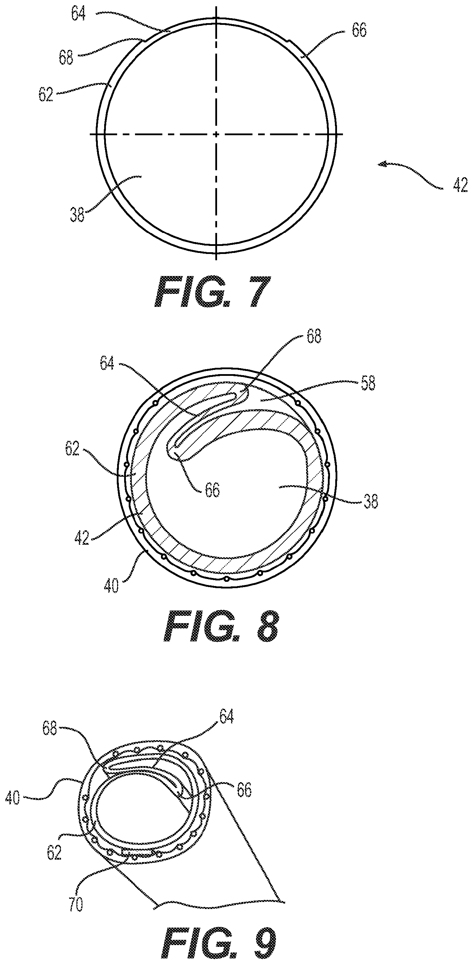

As shown in FIG. 7, the inner tubular layer 42 has a thick wall portion 62 integrally extruded with a thin wall portion 64. The thick wall portion 62 is approximately 0.011 +/- .001 inches 0.02794 +/- 0.00254 cm (0.011 +/- 0.001 inches) and the thin wall portion 66 is approximately 0.01651 +/- 0.00254 cm (0.0065 +/- 0.0010 inches). The inner tubular layer 42 is preferably constructed of a relatively (compared to the outer tubular layer 40) stiff material such as a stiff polymer like high density polyethylene (HDPE) or an equivalent polymer. Integral construction, such as integral extrusion, of the wall portions advantageously avoids the leakage of prior-art sheaths that use a split in the sheath to promote expandability. Prior-art C-sheaths tend to leak close to the proximal end at the manifold where the sheath is stretched the most. Also, integral construction improves the ability to torque the sheath 8.

The thick wall portion 62, in the illustrated embodiment of FIG. 7, has a C-shaped cross section with a first longitudinally extending end 66 and a second longitudinally extending end 68. The ends are where the thickness of the thick wall portion 62 starts to narrow to thin portion 64 on the cross-section. That transition extends longitudinally in the direction of the axis of the sheath 8, such that the thick wall portion 62 forms an elongate C-shaped channel.

From those ends 66, 68 of the thick wall portion 62 extends the thin wall portion 64 and together they define a tubular shape. Extending longitudinally in that tubular shape is the central lumen 38. FIG. 7, in particular, shows the central lumen 38 in its expanded diameter which is larger than the initial diameter of the elastic outer tubular layer 40. For example, the inner tubular layer 42 has a central lumen 38 that is about 0.762 +/- 0.01016 cm (0.300 +/-0.004 inches). The outer tubular layer 40 has an initial elastic lumen 58 of about 0.4699 cm (0.185 inches).

FIGS. 8 and 9 show the inner tubular layer 42 in its compressed or folded condition, folded up and fit into the initial elastic lumen 58 of the outer tubular layer. In the compressed condition, the elastic outer tubular layer 40 urges the first longitudinally extending end 66 under the second longitudinally extending end 68 of the inner tubular layer 42. This positions the thin wall portion 64 between the first and second longitudinally extending ends 66, 68.

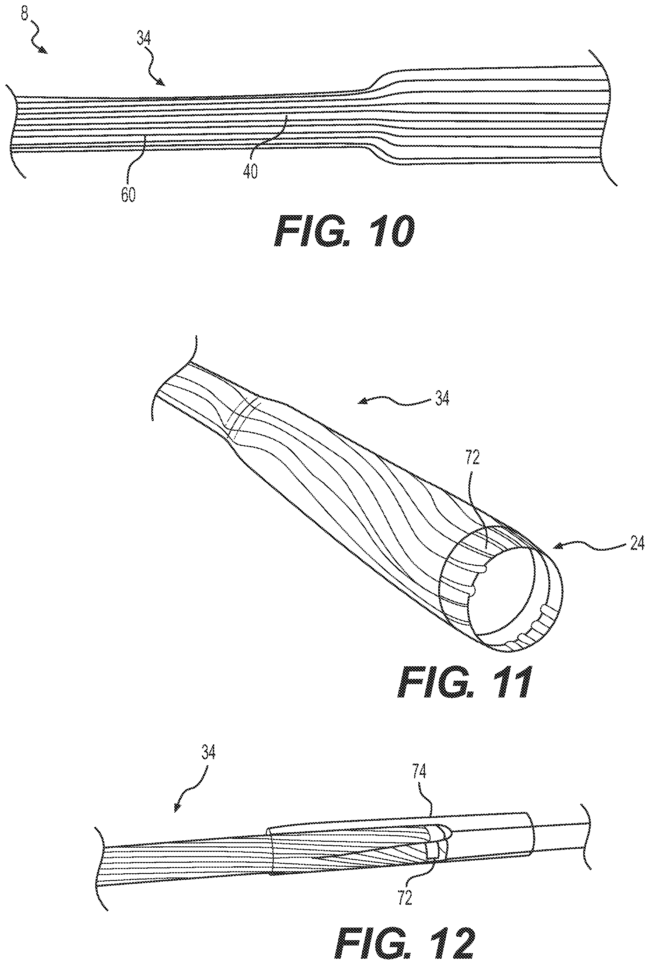

FIG. 10 shows a side view of an implant moving through sheath 8. During passage of an implant through the central lumen 38, the tubular wall structure 34 takes on a locally expanded condition corresponding to the length and geometry of the implant 12. In the expanded condition, the first and second longitudinally extending ends 66, 68 radially expand apart, against the urging of the elastic outer tubular layer 40 by passage of the implant 12, into a non-overlapping condition with the thin wall portion 64 extending therebetween to form the expanded lumen, as in FIG. 7. After passage of the implant 12, the inner tubular layer 42 is urged by the outer elastic tubular layer 40 into the compressed condition shown in FIGS. 8 and 9. With this configuration, a 14 French sheath 8 allows passage of a 29 mm transcatheter heart valve, such as the Sapien XT and Sapien 3 transcatheter heart valves available from Edwards Lifesciences.

As another option, the inner tubular layer 42 may be adhered along one or more longitudinally extending portions of the outer tubular layer 40. Adhesion may be by heat fusion between the two layers or adhesive bonding, for example. As shown in FIG. 9, the longitudinally extending portion can be a strip 70 where the outer surface of the inner tubular layer 42 is bonded or otherwise adhered to the inner surface of the outer tubular layer 40. Preferably, the strip 70 is positioned opposite the thin wall portion 64 to be away from, and not affect, the fold of the inner tubular layer 42. Inhibiting folding would also raise the push force for passage of the implant 12. Another implementation may include a second thin bonding strip 70 or line. Although the thickness of the strip 70 can vary, preferably it is relatively narrow to reduce its inhibition of expansion of the two layers and any increases in pushing force. Use of a narrow bonding line between the layers 40, 42 prevents free rotation of the layers with respect to each other while minimizing the effect on push force.

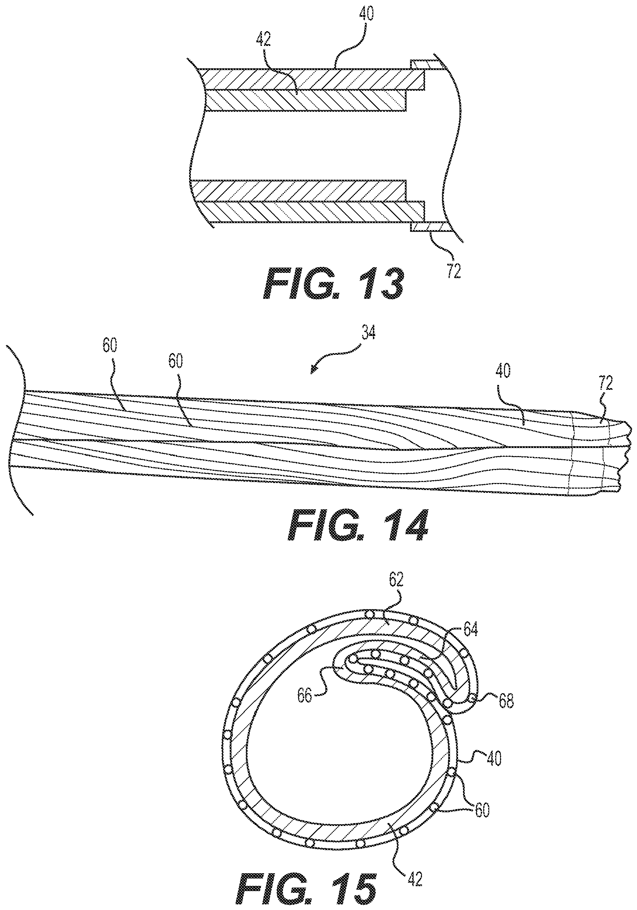

In another embodiment, as shown in FIGS. 11-15, the distal tip 24 of sheath tubular wall structure 34 can be a sealed tip to mitigate blood intrusion and/or facilitate expansion at the distal end of travel of the implant 12. In one aspect, a distal portion of the tubular wall structure 34 may be reflowed to adhere the inner and outer layers 40, 42, as shown in FIG. 11. In particular, the two layers 40, 42 are urged into their fully expanded (unfolded condition) and then reflowed to bind the outer surface of the inner tubular layer 42 to the inner surface of the outer tubular layer 40. Then, the reflowed portion is returned to the compressed or folded configuration and compressed under a heat shrink layer 74 to set the fold. The heat shrink layer 74 is then removed. Thus, when the distal end of the wall structure 34 folds, the outer tubular layer 40 is also folded, as shown in FIGS. 14 and 15. Sealing the tip stops blood from getting between the two layers 40, 42 at the distal end of the sheath 8 while maintaining the highly expandable performance of the tubular wall structure 34.

The reflowed outer tubular layer 40 may have added thereto a radiopaque ring 72. The radiopaque ring 72 can be adhered outside (such as by heat shrinking) and around the reflowed, folded distal portion of the outer tubular layer 40. The ring 72 may be applied (such as by reflowing) outside the outer tubular layer 40 (FIG. 13) or inside the outer tubular layer 40 (FIG. 12). The ring 72 is preferably constructed of a highly elastic polymer to allow expansion and facilitate urging the tip back into a folded configuration.

Advantageously, the outer tubular layer 40 and inner tubular layer 42 are both seamless, which stops blood leakage into the sheath 8. The seamless construction of the inner tubular layer 42 eliminates the ends of a conventional C-sheath. Elimination of the cut in the C-sheath by addition of thin portion 64 improves torque performance. Also, both layers are easily manufactured by an extrusion process. The elastic outer tubular layer 40 has an elastic material that is similar to or the same as most soft tips, making their attachment much easier.

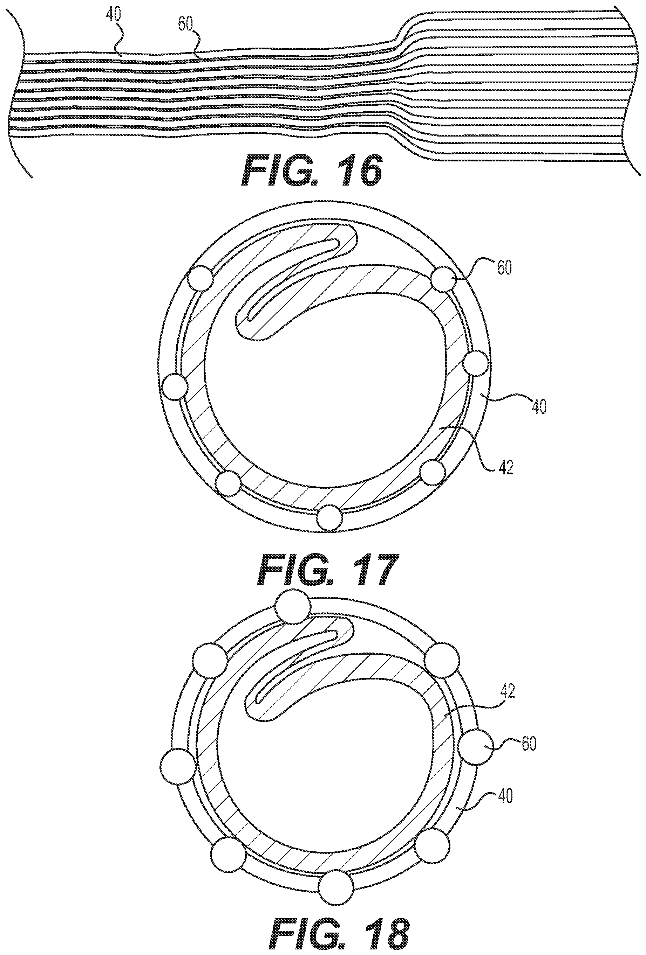

As shown in FIGS. 17-20, other embodiments of the sheath 8 may include a conventional C-shaped inner tubular layer 42 surrounded by an elastic outer tubular layer 40 employing longitudinal rods 60. (FIGS. 17-20 may also use other types of inner tubular layer 42, such as the integrally formed ones disclosed herein.) FIG. 17 shows use of seven longitudinal rods equally spaced from each other about the interior surface of the outer tubular layer 40 with the exception that a rod is missing from a portion adjacent a split in the inner tubular layer 42. This gap facilitates distraction and return of the free edges of the C-shaped inner tubular layer 42. FIG. 18 shows a similar arrangement but with the eighth longitudinal rod 60 present. But the rod is somewhat offset from the location of the free edges of the inner tubular layer 42. Furthermore, the rods of FIG. 18 protrude outward from the outer surface of the outer tubular layer 40 to lower friction between the sheath and, for example, a body lumen or an additional outer delivery sheath.

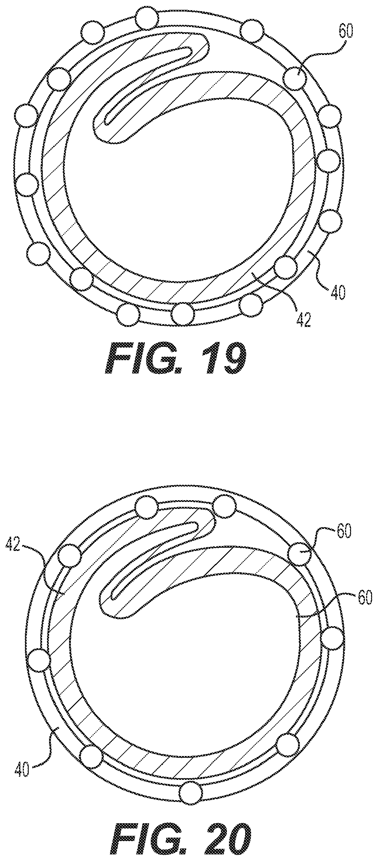

FIG. 19 shows another embodiment wherein rods are embedded in the outer tubular layer 40 and extend from the inside and outside surfaces thereof in alternation. This can lower friction from advancement of the sheath 8 wherein, for example, the outer surface of the layer 40 touches a body lumen or additional outer delivery sheath. FIG. 20 shows another embodiment wherein the inner tubular layer 42 also includes a plurality of longitudinal rods 60 that facilitate, for example, easy passage of the implant 12.

The outer tubular layer 40 in the configurations of FIGS. 17-20 still can have a highly elastic, thin structure to fit over the conventional C-sheath inner tubular layer 42. As the outer tubular layer 40 is not adhered to the inner tubular layer 42, there is free movement between the sleeve and the delivery catheter 10. The outer tubular layer 40 is also seamless to guard against blood leakage. The sheath 8 is stretched evenly along all segments in a radial direction - reducing the risk of tearing or fracture. And, the elastic outer tubular layer 40 will urge the C-shaped sheath back into the reduced profile configuration. During construction, the inner layer 42 is easily fitted inside the outer layer 40 without flattening or heat wrapping. Implementations may include a large number of longitudinal rods 60 - even 100 or more depending upon their cross-sectional size. The longitudinal rods 60 may include microstructure patterns that further reduce friction.

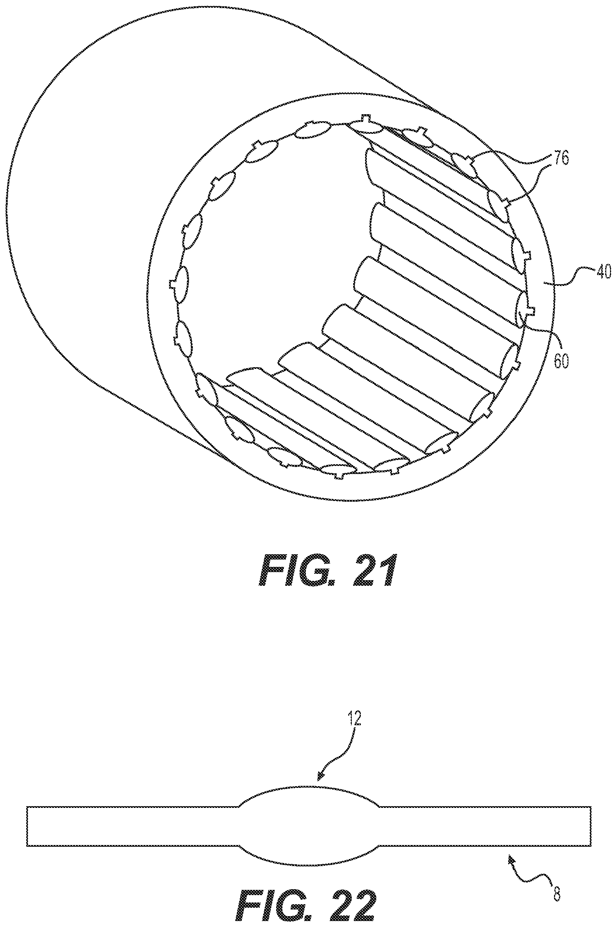

FIGS. 21 and 22 show yet another embodiment of the sheath 8 including a segmented outer tubular layer 40 having longitudinal rods 60 that may be employed with or without an inner tubular layer 42. As shown in FIG. 21, the outer tubular layer 40 has elongate cuts or grooves that form elongate segments 76 extending axially along inner surface. Formed or mounted along the grooves are the longitudinal rods 60. The longitudinal rods 60 are shown in FIG. 21 to have curved or arc-shaped top surfaces that reduce friction for passing implants 12. The longitudinal rods 60 are comprised of relatively high stiffness materials such as HDPE, fluropolymer and PTFE. The outer tubular layer 40 can be constructed of highly elastic materials with a low tensile set (TPE, SBR, silicone, etc.) to facilitate recovery after expansion. When used without an inner tubular layer 42, the outer tubular layer 40 can have additionally lowered expansion force - especially because the higher strength material (the rods) are not connected in the radial direction. Other variations may include changing the number and shape of the rods 60, incorporation of a tie layer or undercut/bard to strengthen the connection of the rods to the outer layer 40 and adding sections of stiff material to the outside of the outer layer for improved stiffness and pushability. A slip additive may be applied to the surfaces to increase lubricity. FIG. 22 shows the bulge in the sheath 8 as the implant 12 passes therethrough.

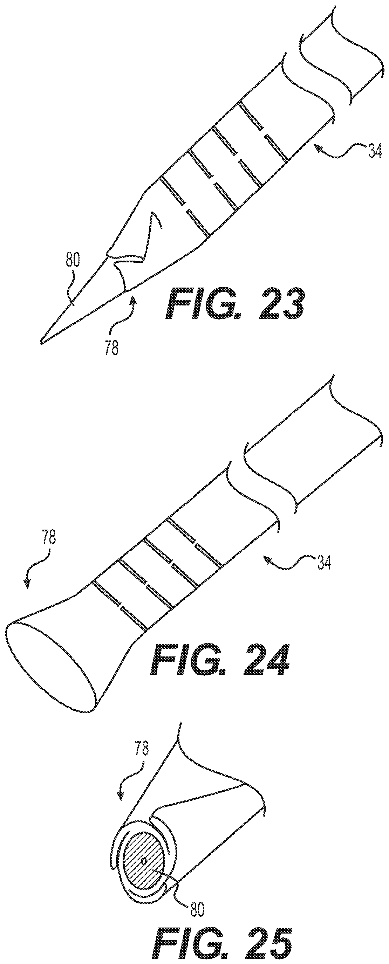

FIGS. 23-25 show another embodiment wherein a distal end of the tubular wall structure 34 can have a flared portion 78. The flared shape of the flared portion 78 helps to reduce snags or interference during retrieval experienced with conventional sheaths during retrieval of medical devices. The flared portion 78 is folded or wrapped around the tapered distal end of an introducer 80 to maintain a low profile for advancement, as shown in FIGS. 23 and 25. The number and size of the folds may vary depending upon the size and material type of the tubular wall structure 34. For example, FIG. 25 shows three folds in a cross-sectional view. After the distal end of the sheath 8 is in position, the introducer 80 is removed. Then, the sheath 8 is ready to receive the delivery catheter 10 and implant 12. When the implant 12 reaches the flared portion 78 the folds then break and expand into the flared configuration, as shown in FIG. 24. The flared portion 78 remains in this flared configuration for possible retrieval of the implant 12.

FIGS. 26-29 show another embodiment of the sheath 8. The sheath 8 includes the tubular wall structure 34 that extends from the proximal end (as shown in cross-section in FIG. 27) to the distal end (FIGS. 28 and 29). Generally, the tubular wall structure 34 includes inner tubular layer 42, inner tip layer 81, strain relief tubular layer 82, outer tip layer 84 and the elastic outer tubular layer 40.

As can be seen the tubular wall structure 34 has different layers depending up on the axial position. The wall structure 34 includes a strain relief tubular layer 82 that terminates about 2/3 of the way from the proximal end, as shown in FIG. 27. The strain relief layer 82 is preferable comprised of a relatively stiff material, such as HDPE, that can withstand the strains of the proximal end of the sheath 8 where it is joined to the hub and 20 and other components for accepting initial insertion of the delivery apparatus 10. It terminates short of the distal end of the sheath 8 to facilitate a greater flexibility and lower profile of the distal end of the sheath 8.

Extending past the strain relief tubular layer 82 the tubular wall structure 34 drops down to two layers, the inner tubular layer 42 and elastic outer tubular layer 40. On the proximal-most end of the portion of the sheath 8 shown in FIG. 27, the inner tubular layer splits (in cross-section) into its thick wall portion 62 and thin wall portion 64 in the folded over configuration.

At the distal end, as shown in FIGS. 28 and 29, the sheath 8 includes tip structure (including inner tip layer 81 an outer tip layer 84) configured to taper the wall structure 34 and seal the free end of the layers against blood or fluid invasion. Generally, these components build up the diameter of a length of the wall structure 34 with some additional layers including stiffening layers, and then tapers out and over the distal free end of the inner tubular layer 42.

The inner tubular layer 42 is similar to that described above. It includes the thin wall portion 64 that is configured to fold over into the folded configuration back onto the thick wall portion 62. Also, the elastic outer tubular layer 40 restrains the inner tubular layer 42 against expansion. But, the elasticity of the outer tubular layer 40 can also be overcome to allow the inner tubular layer to at least partially unfold into a wider central lumen 38 for passage of the implant 12 or other device.

As shown in FIG. 28, the inner tip layer 81 extends only a short axial length. In particular, the inner tip layer 81 extends around and past the distal-most end of the foldable inner tubular layer 42, tapering into smaller diameter free end after extending distally past the free end of the foldable inner tubular layer. As shown in the cross-section orthogonal to the long axis of the sheath 8 of FIG. 29, the inner tip layer 81 has a C-shaped cross-section. (The top of the C-shape is enlarged somewhat to account for the overlapping layers of the wall structure 34 - so that the free longitudinal edges are radially spaced apart to form a gap.) The C-shaped cross-section allows the free longitudinal edges of the inner tip layer 81 to spread apart during unfolding of the inner tubular layer 42. Advantageously, the inner tubular layer 42 has a relatively stiff material construction smoothing, stiffening and tapering the distal end of the sheath 8 as well as providing some protection for the free end of the inner tubular layer 42. The inner tip layer 81 also advantageously extends over the distal end of the inner tubular layer 42, thereby sealing the thick and thin wall portions 62, 64 against blood and fluid invasion.

The outer tip layer 84 extends over and is adhered to the inner tip layer 81 and a distal portion of the inner tubular layer 42. The outer tip layer 84 covers the proximal edge of the inner tip layer 81, sealing it against the inner tubular layer 42. The outer tip layer 84 is of a relatively bendable material and, where it is directly adhered to the thin wall portion 64, can be folded over onto itself as shown in FIG. 28. Advantageously, then, the outer tip layer 84 tracks the unfolding of the thick and thin wall portions 62, 64 to continue to seal the inner tip 81 to the inner tubular layer 42. Notably, as the outer tip layer 84 unfolds the free longitudinal edges of the C-shaped inner tip layer 81 can come apart for coordinated lumen expansion of the sheath 8. But, also, at the same time the stiffness of the inner tip layer 81 and extra reinforcement of the outer tip layer 84 help to maintain tip stiffness and stability.

The elastic outer tubular layer 40 extends all the way to the distal end of the sheath 8, including over the distal end of the outer tip layer 84. In addition, the inside of the elastic outer tubular layer includes rods 60 extending axially and reducing unfolding resistance by lowering surface area and increasing lubricity.

The sheath 8 may also include a radiopaque marker band or layer portion 86 that provides an orientation and depth indication under radioscopy during implantation or other medical procedures.

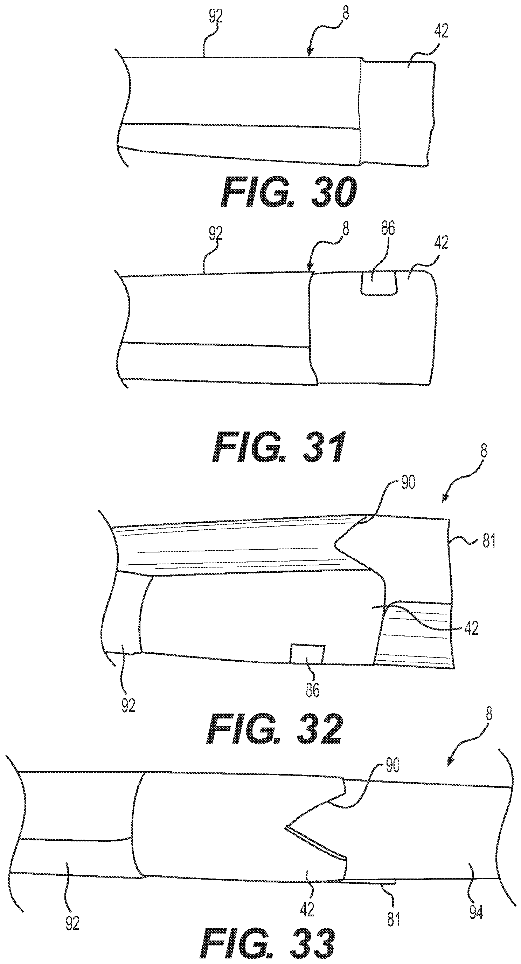

FIGS. 30 through 38 show a method of assembling a stiffened and sealed tip for another embodiment of the sheath 8. FIGS. 30-38 show varying views of the same sheath 8 as it undergoes the method of assembly. FIGS. 30 and 31 show the inner tubular layer 42 (to the right) in the unfolded configuration. An additional tubular layer 92 (such as a strain relief or elastic layer) (to the left) extends over the inner tubular layer 42 but stops short of the free end of the inner tubular layer. FIG. 31 shows a portion of the radiopaque marker 86 attached to the inner tubular layer 42.

FIG. 32 shows the inner tubular layer 42 with a window or v-shaped notch 90 cut into its free end to allow for tip expansion. The v-shaped notch 90 also facilitates retrieval of an implant. FIG. 32 also shows the C-shaped inner tip layer 81 extended around an outside of the inner tubular layer. FIG. 33 shows a second notch 90 on the opposite side of the inner tubular layer 42. Also in FIG. 33, the distal tip of the partially constructed sheath 8 is extended over a mandrel 94 to facilitate folding and attachment of other layers.

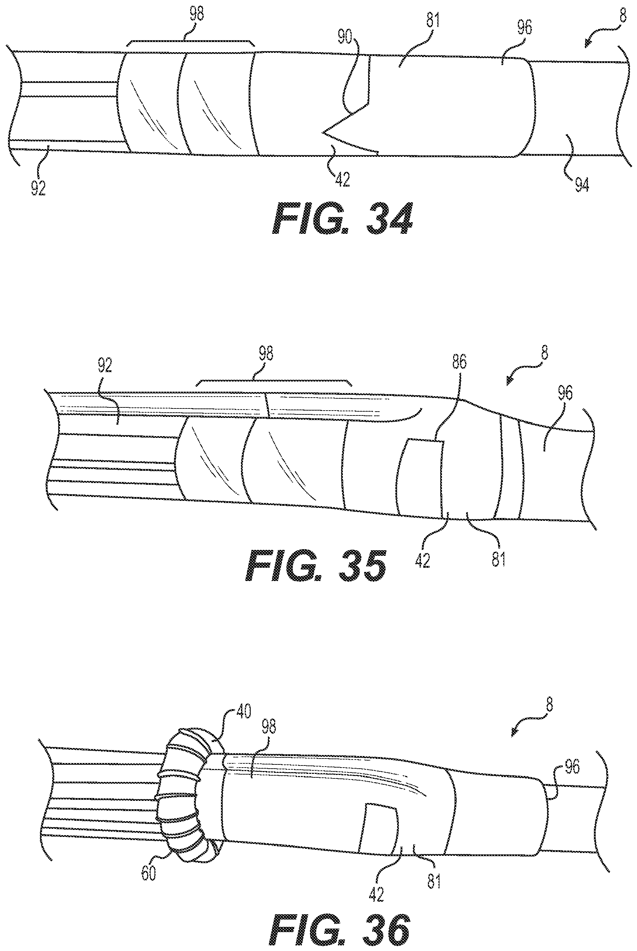

FIG. 34 shows formation of a proximal hemostasis seal by application of a proximal sealing layer 96 that extends around a distal free end of the additional tubular layer 92 and over and past the distal end of the emerging inner tubular layer 42. In the embodiment shown in FIG. 34, the proximal sealing layer 96 is transparent such that the v-shaped notch 90 is visible from underneath the sealing layer 96. A proximal section 98 of the sealing layer 96 is heat treated to seal the transition between the additional tubular layer 92 and the inner tubular layer 42, which in some embodiments can give proximal section 98 a glossier appearance than the remainder of sealing layer 96. The proximal section 98 blocks blood and other fluids from entering between the two layers 42, 92.

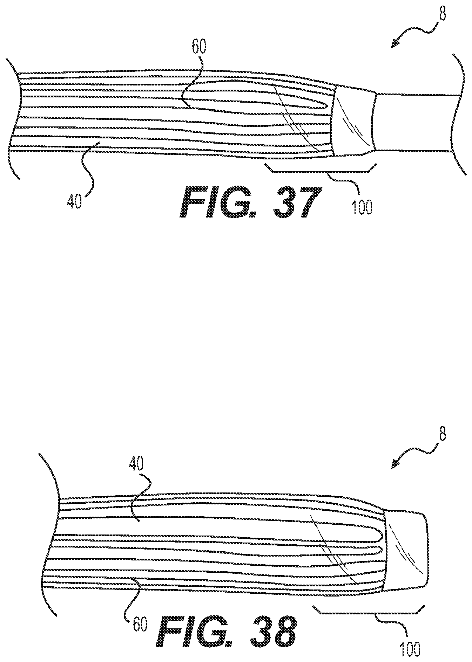

FIG. 35 shows the layers 42, 92 and 96 being folded over onto themselves. FIG. 36 shows the elastic outer tubular layer 40 or jacket with rods 60 being unrolled over the now folded layers 42, 92 and 96. FIG. 37 shows the outer tubular layer 40 itself slightly folded at the distal end and having applied thereover a distal sealing layer 100. The excess of the free end of the proximal sealing layer 96 extending past the distal sealing layer 100 is cut away. The distal sealing layer advantageously urges the distal free end of the layers 40, 42 and 96 into a tapered configuration and provides a rounded distal end for the tubular wall structure 34 that facilitates insertion and advancement over the guidewire.

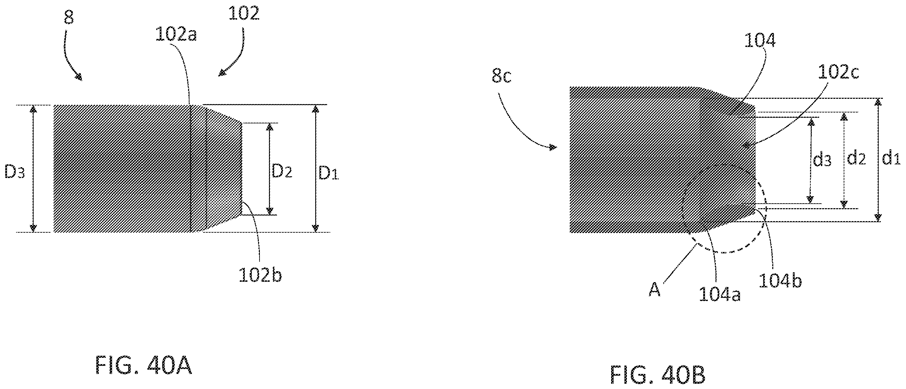

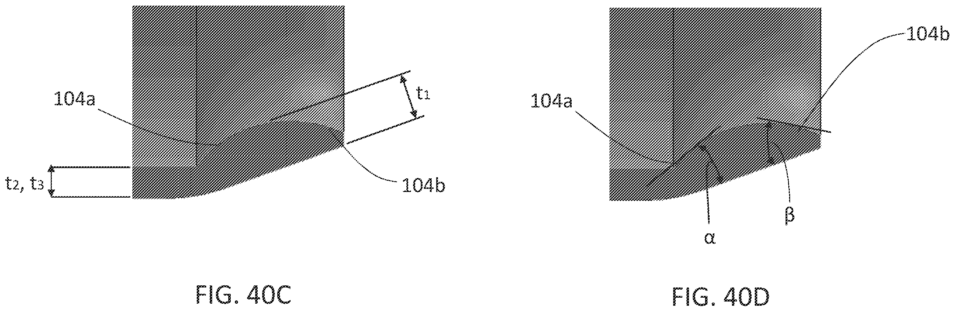

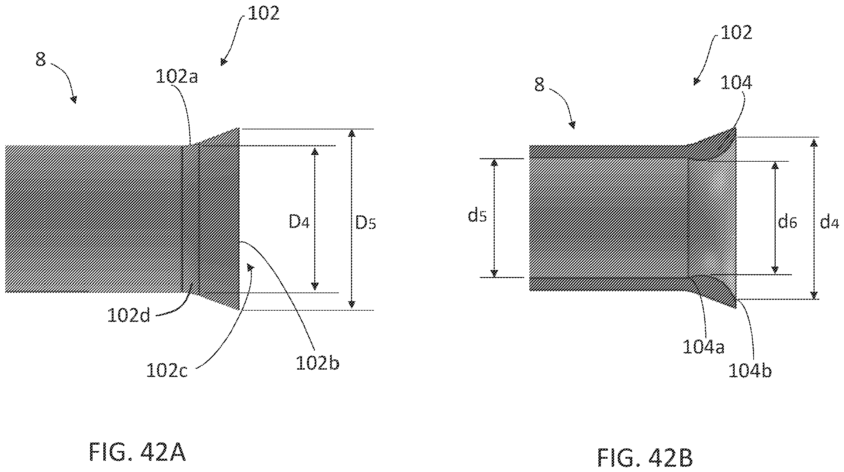

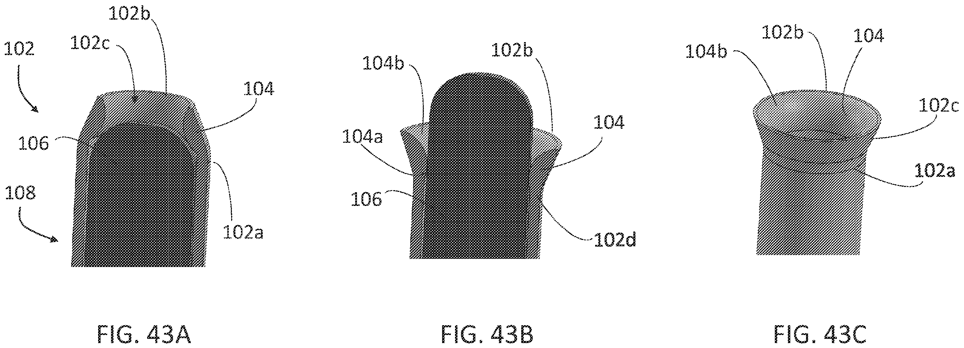

FIGS. 39-43 illustrate another embodiment of the sheath 8 of the introducer sheath system. As provided in FIG. 39, the sheath 8 includes a tubular wall structure 34 that extends from the proximal end 8a to the distal end 8b of the sheath 8, with a central lumen extending therethrough. An expandable tip portion 102 is provided at the distal end 8b of the sheath 8. The distal end of the tip portion 102 includes a tapered outer surface that terminates at the distal opening of the sheath 8. The tapered outer surface helps to reduce trauma when the sheath 8 enters the patient and is moved through the blood vessel or other patient anatomy. As will be described in more detail below, the tip portion 102 is movable between a non-expanded configuration (FIGS. 40-41) and expanded configuration (FIG. 42). As illustrated in FIGS. 42B and 43B, in the expanded configuration, both the interior and exterior surfaces of the tip portion 102 flare into a funnel-like shape during passage of an implant or other device therethrough (as used herein, "implant" and/or "payload" refer to any implant (e.g., valve, stent, or other prosthetic device), balloon, dilator, medical instrument or tool, sheath, or any other device or tool delivered to a patient's vascular or non-vascular tissue via the sheath 8). The flared funnel-shaped structure of the expanded tip portion 102 facilitates retrieval of the implant/device and reduces trauma to the patient tissue and damage the implant as the implant is withdrawn back into the sheath 8.

FIGS. 40A-40B and 42A-42B provide side views of a portion of the distal end of the sheath 8 including the tip portion 102. In both the expanded and non-expanded configurations, the tip portion 102 defines a tubular structure between the proximal end 102a and the distal end 102b of the tip portion 102, and a central lumen 102c extending therethrough. The central lumen 8c of the sheath 8 forms a continuous central lumen with the central lumen 102c of the tip portion 102, see e.g., FIG. 40B. For example, as illustrated in FIG. 40B, at the proximal end 102a of the tip portion 102, the central lumen 102c forms a passage having a uniform diameter with the central lumen 8c of the sheath 8.

As illustrated in FIG. 40A, when the tip portion 102 is in an unexpanded configuration, the outer diameter of the tip portion 102 decreases between the proximal and distal ends 102a, 102b of the tip portion 102. As such the outer diameter (D₁) of the proximal end 102a of the tip portion 102 and the sheath 8 is greater than the outer diameter (D₂) of the distal end 102b of the tip portion 102, thereby forming a sloped and/or tapered outer surface at the distal end of the sheath 8. As provided in FIG. 40A, the outer diameter (D3) of the sheath corresponds to the outer diameter (D₁) of the proximal end 102a of the tip portion 102. In another embodiment (not shown), the outer diameter (D₃) of the sheath 8 is greater than the outer diameter (D₁) of the tip portion 102, such that the transition between the sheath 8 and the tip portion 102 is defined by decreasing tapered or stepped outer surface of the sheath 8/tip portion 102. In another embodiment (not shown), the outer diameter (D₃) of the sheath 8 is less than the outer diameter (D₁) of the tip portion 102, such that the transition between the sheath 8 and the tip portion 102 is defined by increasing tapered or stepped outer surface of the sheath 8/tip portion 102.

As illustrated in FIG. 40B, in the unexpanded configuration, the diameter (d₁) of the central lumen 102c at a proximal end 102a of the tip portion 102 is greater than the opening diameter (d₂) of the central lumen 102c at the distal end 102b of the tip portion 102. The camming feature 104 is provided on an interior surface of the central lumen 102c of the tip portion 102. The camming feature 104 is formed as an inward protrusion from the interior surface of the tip portion 102 projecting into the central lumen 102c towards the longitudinal axis of the tip portion 102/sheath 8. In an example embodiment, the inner most diameter of the tip portion 102 is defined by the camming feature 104, which extends uniformly about the circumference of the interior surface of the tip portion 102. For example, as illustrated in FIG. 40B, the inner diameter (d3) defined by the camming feature 104 is less than both the opening diameter (d2) of the tip portion 102 and the diameter (d1) of the proximal end 102a. As illustrated in FIG. 40C (providing an enlarged view of Area A of FIG. 40B), the thickness (tl) of the tip portion 102 along the camming feature 104 (e.g., at an apex of the camming feature 104) is greater than the thickness (t2) of the tip portion 102 excluding the camming feature 104 and/or the thickness (t3) of the sheath 8. Also, in some embodiments, as illustrated in FIGS. 40B and 42B, the thickness (tl) of the camming feature 104 with respect to the outer surface of the tip portion 102 remains constant between the non-expanded and expanded configurations. Further, in some embodiments (not shown), the camming feature extends non-uniformly about the circumference of the interior surface of the tip potion 102.

The camming feature 104 includes a leading edge 104a and an adjoining or adjacent trailing edge 104b. The leading edge 104a is adjacent the proximal end 102a of the tip portion 102 and the first portion of the camming feature 104 to engage an implant or other device inserted through/into the sheath 8, see e.g., FIG. 43A. The trailing edge 104b of the camming feature 104 is provided adjacent to the distal end 102b of the tip portion 102. As will be described below, the trailing edge 104b provides a sloped contact surface for the implant/payload as it is withdrawn back through the tip portion 102 and into the sheath 8.

In some embodiments, the leading edge 104a and the trailing edge 104b of the camming feature 104 form a smooth continuous surface angled to facilitate the movement of an implant or other payload therethrough. For example, the camming feature 104 can define a curvilinear surface projecting inward from the interior surface of the central lumen 102c of the tip portion 102. In some embodiments, as illustrated in FIG. 40B, the curvilinear surface is a uniform convex surface projecting inward toward the central lumen 102c of the tip portion 102. In other embodiments, the camming surface 104 forms an asymmetrical convex surface projecting inward toward the central lumen 102c of the tip portion 102. For example, an asymmetrical camming surface 104 may include a portion with a steeper slope/angle on the leading edge 104a than the trailing edge 104b and/or the leading edge 104a may have a radius of curvature different from the radius of curvature of the trailing edge 104b.

In some embodiments, the leading edge 104a has a leading edge slope and the trailing edge 104b has a trailing edge slope. The slope can be measured with respect to the outer tapered surface of the tip portion. For example, as illustrated in FIG. 40D (providing an enlarged view of Area A of FIG. 40B), the angle of the leading edge 104a is indicated by the angle α and the angle of the trailing edge 104b is indicated by the angle β. In some embodiments, the slope (and/or angle α) of the leading edge 104a is less than the slope (and/or angle β) of the trailing edge 104b. In other embodiments, the slope (and/or angle α) of the leading edge 104a is equal to the slope (and/or angle β) of the trailing edge 104b.

As described above, the tip portion 102 is movable between a non-expanded configuration (FIGS. 40A, 41A, 43A) and an expanded configuration (FIGS. 42A, 43B). In some embodiments, for example, the tip portion 102 can bend or otherwise deform at the proximal end 102a to allow the distal end 102b of the tip portion 102 to flare open to the expanded configuration. For example, the proximal end 102a of the tip portion 102 can be formed from an elastomer material, such that elastomer material bends or flexes allowing the tip portion 102 to expand. The elastomer material is formed such that the tip portion 102 is biased to remain in the non-expanded configuration requiring no external force be applied to the camming feature 104. In some embodiments, the tip portion 102 expands radially as a payload 106 (FIGS. 43A and 43B) is advanced through the tip portion 102 such that the central lumen 102c of the tip portion 102 can accommodate payloads of various widths/diameters.

The sheath 8 and the tip portion 102 form a continuous central lumen 8c, 102c, extending between the proximal end 8a of the sheath 8 and the distal end 102b of the tip portion 102. In some embodiments, the sheath 8 and the tip portion 102 are formed as a uniform body. In some embodiments, the tip portion 102 and the sheath 8 are formed as separate bodies and the proximal end 102a of the tip potion 102 is coupled to the distal end 8b of the sheath 8. When the tip portion 102 and sheath 8 are formed as separate components, they may be coupled using any mechanical or chemical fastener suitable for connecting two sheath components including, for example, by soldering, reflow, and/or adhesive. In some embodiments, the sheath 8 is formed from the same elastomer material as the tip portion 102. Whereas, in other embodiments, the sheath 8 and the tip portion 102 are formed from different elastomers. When the sheath 8 and tip portion 102 are formed from different elastomers, the elastomer of the sheath 8 can have a higher elasticity than the elastomer of the tip portion 102.

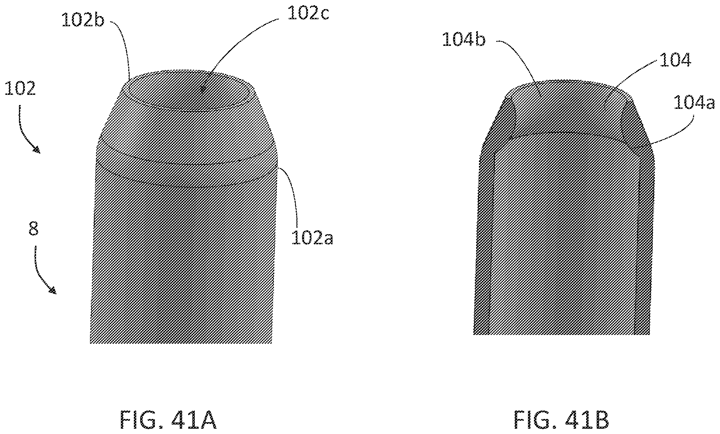

FIGS. 40A-40D and 41A-41B show the tip portion 102 in the non-expanded configuration. As described above, in some embodiments, the tip portion 102 is biased in a non-expanded configuration, such that the tip portion 102 is in the non-expanded configuration when no external force is applied to the camming feature 104 and/or the exterior surface of tip portion 102. In the non-expanded configuration, the outer diameter (D₁) of the proximal end 102a of the tip portion 102 is greater than the outer diameter (D₂) of the distal end 102b of the tip portion 102 such that at least a portion of the outer surface 102d of the tip portion defines a decreasing tapered surface. The smaller diameter of the distal end 102b and the tapered surface are configured to direct the sheath into/through a blood vessel/patient anatomy with minimal trauma to the blood vessel and/or surrounding tissue. This configuration also promotes the gradual expansion of the blood vessel/patient anatomy when the sheath 8 is inserted, limiting the trauma caused by abrupt expansion of the blood vessel walls/tissue.

FIGS. 42A-42B show the tip portion 102 in an expanded configuration. In the expanded configuration, the outer diameter (D₄) of the proximal end 102a of the tip portion 102 is less than or equal to the outer diameter (D₅) of the distal end 102b. As illustrated in FIG. 42B, the proximal end 102a of the tip portion 102 bends or otherwise deforms (e.g., at location 102d) allowing the distal end 102b of the tip portion 102 to flare open forming a funnel-shaped structure when expanded. When in the expanded configuration, the diameter (d₄) of the distal opening/end 102b of the tip portion 102 is greater than the diameter (d₅) of the central lumen of the sheath 8 (D2). The outer surface of the tip portion 102 forms an increasing tapered surface (compared to the outer surface of the sheath 8). The increasing tapered surface promotes the gradual expansion of the blood vessel or patient tissue and helps to prevent trauma.

As illustrated in FIG. 42B, in the expanded configuration, at least a portion of the camming feature 104 defines an internal diameter (d₆) less than the diameter (d₅) of the central lumen of the sheath 8. Accordingly, when the tip portion 102 is in the expanded configuration, an implant/payload exiting the sheath 8 is directed toward the center of the tip portion 102 via contact with the camming feature 104. The funnel-like structure formed by the internal surface of the tip portion 102/camming feature 104 also discourages the implant/payload from advancing in a direction away from the longitudinal axis of the sheath during delivery, e.g., towards the blood vessel walls.

FIGS. 43A-43B show an example implant/payload 106 provided within the sheath 8/tip portion 102 moving the tip portion 102 from the non-expanded configuration (FIG. 43A) to the expanded configuration (FIG. 43B). FIG. 43C illustrates a perspective view of the tip portion 102 in an expanded configuration without the implant/payload 106. As described above, the sheath 8 is introduced into a blood vessel or other patient anatomy, to create a passage for an implant/payload 106 to be advanced into the blood vessel through the sheath 8. Typically, an obturator is inserted into the sheath 8 prior to introduction of any medical device to assist in placement of the sheath 8 at the desired location within the patient. To reduce trauma to the patient tissue, it is desirable that the sheath 8 is advanced while the sheath tip 102 is in the non-expanded configuration. Accordingly, the obturator can include a tubular member adapted to slideably insert into the central lumen of the sheath 8 (and central lumen 102c of the tip portion 102) without expanding the tip portion 102. The distal end of the obturator may extend past the distal opening/distal end 102b of the tip portion 102, and the sheath 8 can be advanced into position within the patient. Once positioned, the proximal end of the sheath 8 may be secured to the body tissue, e.g., by sutures. The obturator is then removed from the central lumen of the sheath 8 and the implant/payload 106 and/or other medical device advanced through the sheath 8 and into position within the patient.

With the sheath 8 in place, the implant/payload 106 is advanced through the proximal end 8a of the sheath 8 to the proximal end 102a of the tip portion 102. As an illustrative example, FIG 43A shows the sheath 8 and the tip portion 102 in the non-expanded configuration. A payload 106 is advanced through the sheath 8 up to the tip portion 102 of the sheath 8. As illustrated in FIG. 43B, as the payload 106 is further advanced through the sheath 8/tip portion 102, the tip portion 102 transitions from the non-expanded configuration to the expanded configuration. As the payload 106 advances through the sheath 8/tip portion 102, the distal end and side surfaces of the payload 106 engage the camming feature 104 pushing against the leading edge 104a. The camming feature 104 is connected to the remainder of the tip portion 102 such that the force against the leading edge 104a causes (at least a portion of) the proximal end 102a of the tip portion 102 to bend or otherwise deform, for example, at location 102d. As the camming feature 104 rotates away from the longitudinal axis of the sheath 8 and the distal end 102b of the tip portion 102 flares outward.

As shown in FIG. 43B, at least part of the payload 106 is extended beyond the distal opening/distal end 102b of the tip portion 102, which is still in the expanded configuration. In some embodiments, at least part of the payload 106 may be withdrawn back through the distal end 102b of the tip portion 102 toward the sheath 8 (e.g., during retrieval/repositioning of an implant). As described above, the funnel-like structure of the internal surface of the tip portion 102/camming feature 104 also facilitates retrieval/withdraw of the implant/payload 106 during removal and/or repositioning. For example, as illustrated in FIG. 43B, the trailing edge 104b of the camming feature 104 forms a smooth tapered surface that directs the implant/payload back into the sheath 8 with minimal applied force. Also, in the case of an expandable implant/payload 106, the funnel-like structure encourages compression and/or folding of the implant/payload 106, reducing damage to the payload 106 and providing more efficient contraction to the reduced diameter of the sheath 8. It is contemplated that the trailing edge 104b may include projections/ridges and/or grooves/channels that facilitate folding/contraction of the payload 106 into a certain configuration.

In some embodiments, the tip portion 102 is biased to return to the non-expanded configuration when the payload 106 force is removed from the camming feature 104. Accordingly, the tip portion 102 only remains in the expanded configuration while the camming feature 104 is engaged. As the payload 106 is withdrawn through the tip portion 102, the force applied against the leading edge 104a (and/or any other portion of the camming feature 104) is removed from the camming feature 104, and the tip portion 102 moves from the expanded configuration to the non-expanded configuration. Return to the non-expanded configuration is facilitated, at least in part, due to the bias in the elastomer material provided at the proximal end 102a of the tip portion 102. In some embodiments, the payload 106 is withdrawn from the tip portion 102 and the sheath 8, toward the proximal end 8a of the sheath 8 and remains in the sheath 8 for repositioning. The payload 106 can also be fully removed from the sheath 8 and the tip portion 102 and a second/other payload can be advanced through the sheath 8/tip portion 102 and the tip portion 102 can move between the expanded and non-expanded configuration as described above.

In some embodiments, the payload 106 is a catheter. In some embodiments, the catheter is part of the introducer sheath system and is at least partially disposed within the central lumen of the sheath 8. The outer diameter of the catheter is smaller than the diameter of the central lumen of the sheath 8, such that it slides therethrough. The catheter can be advanced from the proximal end 8a of the sheath 8 toward the distal end 8b of the sheath 8. The catheter can also be advanced from the proximal end 102a of the sheath tip 102 toward and through the distal end 102b of the sheath tip 102. The outer surface of the catheter can engage the camming surface 104 to expand the tip portion 102, as described above with respect to payload 106.

In view of the many possible embodiments to which the principles of the disclosed invention can be applied, it should be recognized that the illustrated embodiments are only preferred examples of the invention. The scope of the invention is defined by the following claims. We therefore claim as our invention all that comes within the scope of these claims.

## Claims

1. An introducer sheath system comprising:
a sheath member (8) defining a tubular structure (34) extending between a proximal end (8a) and a distal end (8b) of the sheath; and
a tip portion (102) provided at the distal end (8b) of the sheath member (8), the tip portion (102) defining a tubular structure extending between a proximal end (102a) and a distal end (102b) of the tip portion (102) and having a central lumen (102c) extending therethrough, the tip portion (102) including a camming feature (104) formed as a protrusion from the central lumen (102c) of the tip portion (102),
wherein the camming feature (104) comprises a leading edge (104a) and an adjoining trailing edge (104b), the leading edge (104a) adjacent the proximal end (102a) of the tip portion (102) and the trailing edge (104b) adjacent the distal end (102b) of the tip portion (102);
wherein the tip portion (102) movable between a non-expanded configuration when the camming feature (104) is not engaged and an expanded configuration when the camming feature (104) is engaged,
wherein, when the tip portion (102) is in the non-expanded configuration, a diameter of the distal end (102b) of the tip portion (102) is less than a diameter of the proximal end (102a) of the tip portion (102); and
wherein, when the tip portion (102) is in the expanded configuration, at least a portion of the camming feature (104) defines an internal diameter (d₆) less than the diameter (d₅) of the central lumen (38) of the sheath (8).

2. The introducer sheath system of claim 1, wherein, when the tip portion (102) is in the non-expanded configuration at least a portion of an outer surface of the tip portion (102) defines a decreasing tapered surface extending between the proximal and distal ends (102a, 102b) of the tip portion (102).

3. The introducer sheath of any one of claims 1 and 2, wherein the sheath member (8) and the tip portion (102) form a continuous central lumen extending between the proximal end (8a) of the sheath member (8) and the distal end (102b) of the tip portion (102).

4. The introducer sheath system of any one of claims 1-3, wherein the camming feature (104) is formed as a curvilinear surface projecting from the central lumen (102c) of the tip portion (102);
wherein the curvilinear surface preferably comprises:
a) a uniform convex surface; or
b) an asymmetrical convex surface.

5. The introducer sheath system of any one of claims 1-4, wherein a slope of the leading edge (104a) is less than a slope of the trailing edge (104b).

6. The introducer sheath system of claim 5, wherein the leading edge (104a) has a radius of curvature that is:
a) the same as a radius of curvature of the trailing edge (104b); or
b) different from a radius of curvature of the trailing edge (104b).

7. The introducer sheath system of any one of claims 1-6, wherein the camming feature (104) extends circumferentially about an interior surface of the tip portion (102),
wherein the shape of the camming feature (104) is preferably:
a) circumferentially uniform about the interior surface of the tip portion (102); or
b) circumferentially non-uniform about the interior surface of the tip portion (102).

8. The introducer sheath system of any one of claims 1-7, wherein an inner most diameter of the tip portion (102) is defined by the camming feature (104),
wherein an inner most diameter of the sheath system is preferably defined by the camming feature (104).

9. The introducer sheath system of any one of claims 5-8, wherein the trailing edge (104b) is sloped to accept an implant (12) or other payload as it is advanced through the distal end (102b) of the tip portion (102) toward the proximal end (102a) of the tip portion (102).

10. The introducer sheath system of any one of claims 1-9, wherein:
a) the camming feature (104) has a smooth continuous surface angled to facilitate the movement of an implant (12) or other payload therethrough; and/or
b) a thickness of the tip portion (102) along the camming feature (104) is greater than a thickness along a portion of the tip portion (102) excluding the camming feature (104).

11. The introducer sheath system of any one of claims 1-10, wherein at least a portion of the tip portion (102) deforms upon transition between the non-expanded and expanded configurations allowing the distal end (102b) of the tip portion (102) to flare open to a larger diameter in the expanded configuration;
wherein a thickness of the camming feature (104) preferably remains constant in both the expanded and non-expanded configuration.

12. The introducer sheath system of any one of claims 1-11, wherein:
a) the tip portion (102) is biased to the non-expanded configuration; and/or
b) when the tip portion (102) is in the expanded configuration, a portion of an outer surface of the tip portion (102) forms an increasing tapered surface.

13. The introducer sheath system of any one of claims 1-12, further comprising a catheter (14), wherein the catheter (14) is at least partially disposed within a central lumen (38) of the sheath member (8) and movable longitudinally therethrough,
wherein the catheter (14) is movable through the central lumen (102c) of the tip portion (102),
wherein engagement between the catheter (14) and the camming feature (104) causes the tip portion (102) to transition from the non-expanded configuration to expanded configuration.

14. The introducer sheath system of any one of claims 1-13, wherein the sheath member (8) is radially expandable.

15. The introducer sheath system of any one of claims 1-14, wherein:
a) the proximal end (102a) of the tip portion (102) is formed from an elastomer material; and/or
b) the sheath member (8) and the tip portion (102) are formed from an elastomer material.

## Patentansprüche

1. Einführerhülsensystem, umfassend:
ein Hülsenelement (8), das eine röhrenförmige Struktur (34) definiert, die sich zwischen einem proximalen Ende (8a) und einem distalen Ende (8b) der Hülse erstreckt; und
einen Spitzenabschnitt (102), der an dem distalen Ende (8b) des Hülsenelements (8) bereitgestellt ist, wobei der Spitzenabschnitt (102) eine röhrenförmige Struktur definiert, die sich zwischen einem proximalen Ende (102a) und einem distalen Ende (102b) des Spitzenabschnitts (102) erstreckt und ein zentrales Lumen (102c) hat, das sich dort hindurch erstreckt, wobei der Spitzenabschnitt (102) ein Nockenmerkmal (104) aufweist, das als Vorsprung von dem zentralen Lumen (102c) des Spitzenabschnitts (102) gebildet ist,
wobei das Nockenmerkmal (104) eine vordere Kante (104a) und eine sich anschließende hintere Kante (104b) umfasst, wobei die vordere Kante (104a) an das proximale Ende (102a) des Spitzenabschnitts (102) und die hintere Kante (104b) an das distale Ende (102b) des Spitzenabschnitts (102) angrenzt;
wobei der Spitzenabschnitt (102) zwischen einer nicht ausgedehnten Auslegung, wenn das Nockenmerkmal (104) nicht im Eingriff ist, und einer ausgedehnten Auslegung, wenn das Nockenmerkmal (104) im Eingriff ist, beweglich ist,
wobei, wenn der Spitzenabschnitt (102) in der nicht ausgedehnten Auslegung ist, ein Durchmesser des distalen Endes (102b) des Spitzenabschnitts (102) kleiner ist als ein Durchmesser des proximalen Endes (102a) des Spitzenabschnitts (102); und
wobei, wenn der Spitzenabschnitt (102) in der ausgedehnten Auslegung ist, mindestens ein Abschnitt des Nockenmerkmals (104) einen Innendurchmesser (d₆) definiert, der kleiner ist als der Durchmesser (d₅) des zentralen Lumens (38) der Hülse (8).

2. Einführerhülsensystem nach Anspruch 1, wobei, wenn der Spitzenabschnitt (102) in der nicht ausgedehnten Auslegung ist, mindestens ein Abschnitt einer Außenseite des Spitzenabschnitts (102) eine abnehmende konische Fläche definiert, die sich zwischen dem proximalen und dem distalen Ende (102a, 102b) des Spitzenabschnitts (102) erstreckt.

3. Einführerhülsensystem nach einem der Ansprüche 1 und 2, wobei das Hülsenelement (8) und der Spitzenabschnitt (102) ein durchgängiges zentrales Lumen bilden, das sich zwischen dem proximalen Ende (8a) des Hülsenelements (8) und dem distalen Ende (102b) des Spitzenabschnitts (102) erstreckt.

4. Einführerhülsensystem nach einem der Ansprüche 1-3, wobei das Nockenmerkmal (104) als eine gekrümmte Oberfläche gebildet ist, die von dem zentralen Lumen (102c) des Spitzenabschnitts (102) vorsteht;
wobei die gekrümmte Oberfläche vorzugsweise umfasst:
a) eine gleichmäßige konvexe Oberfläche; oder
b) eine asymmetrische konvexe Oberfläche.

5. Einführerhülsensystem nach einem der Ansprüche 1-4, wobei eine Neigung der vorderen Kante (104a) kleiner ist als eine Neigung der hinteren Kante (104b).

6. Einführerhülsensystem nach Anspruch 5, wobei die vordere Kante (104a) einen Krümmungsradius hat, der:
a) derselbe ist wie ein Krümmungsradius der hinteren Kante (104b); oder
b) sich von einem Krümmungsradius der hinteren Kante (104b) unterscheidet.

7. Einführerhülsensystem nach einem der Ansprüche 1-6, wobei sich das Nockenmerkmal (104) in Umfangsrichtung um eine Innenfläche des Spitzenabschnitts (102) erstreckt, wobei die Form des Nockenmerkmals (104) vorzugsweise:
a) in Umfangsrichtung gleichmäßig um die Innenfläche des Spitzenabschnitts (102) verläuft; oder
b) in Umfangsrichtung nicht gleichmäßig um die Innenfläche des Spitzenabschnitts (102) verläuft.

8. Einführerhülsensystem nach einem der Ansprüche 1-7, wobei ein innerster Durchmesser des Spitzenabschnitts (102) durch das Nockenmerkmal (104) definiert wird, wobei ein innerster Durchmesser des Hülsensystems vorzugsweise durch das Nockenmerkmal (104) definiert wird.

9. Einführerhülsensystem nach einem der Ansprüche 5-8, wobei die hintere Kante (104b) geneigt ist, um ein Implantat (12) oder eine andere Nutzlast aufzunehmen, wenn es bzw. sie durch das distale Ende (102b) des Spitzenabschnitts (102) in Richtung des proximalen Endes (102a) des Spitzenabschnitts (102) vorgeschoben wird.

10. Einführerhülsensystem nach einem der Ansprüche 1-9, wobei:
a) das Nockenmerkmal (104) eine glatte durchgehende Oberfläche hat, die abgewinkelt ist, um die Bewegung eines Implantats (12) oder einer anderen Nutzlast dort hindurch zu erleichtern; und/oder
b) eine Dicke des Spitzenabschnitts (102) entlang des Nockenmerkmals (104) größer ist als eine Dicke entlang eines Abschnitts des Spitzenabschnitts (102), ohne das Nockenmerkmal (104).

11. Einführerhülsensystem nach einem der Ansprüche 1-10, wobei sich mindestens ein Abschnitt des Spitzenabschnitts (102) beim Wechsel zwischen der nicht ausgedehnten und der ausgedehnten Auslegung verformt, was es dem distalen Ende (102b) des Spitzenabschnitts (102) ermöglicht, sich in der ausgedehnten Auslegung auf einen größeren Durchmesser zu öffnen;
wobei eine Dicke des Nockenmerkmals (104) sowohl in der ausgedehnten als auch in der nicht ausgedehnten Auslegung vorzugsweise konstant bleibt.

12. Einführerhülsensystem nach einem der Ansprüche 1-11, wobei:
a) der Spitzenabschnitt (102) auf die nicht ausgedehnte Auslegung vorgespannt ist; und/oder
b) wenn der Spitzenabschnitt (102) in der ausgedehnten Auslegung ist, ein Abschnitt einer Außenseite des Spitzenabschnitts (102) eine zunehmende konische Oberfläche bildet.

13. Einführerhülsensystem nach einem der Ansprüche 1-12, ferner umfassend einen Katheter (14), wobei der Katheter (14) mindestens teilweise in einem zentralen Lumen (38) des Hülsenelements (8) angeordnet und in Längsrichtung dort hindurch beweglich ist,
wobei der Katheter (14) durch das zentrale Lumen (102c) des Spitzenabschnitts (102) beweglich ist,
wobei der Eingriff zwischen dem Katheter (14) und dem Nockenmerkmal (104) bewirkt, dass der Spitzenabschnitt (102) aus der nicht ausgedehnten Auslegung in die ausgedehnte Auslegung wechselt.

14. Einführerhülsensystem nach einem der Ansprüche 1-13, wobei das Hülsenelement (8) radial ausdehnbar ist.

15. Einführerhülsensystem nach einem der Ansprüche 1-14, wobei:
a) das proximale Ende (102a) des Spitzenabschnitts (102) aus einem Elastomermaterial gebildet ist; und/oder
b) das Hülsenelement (8) und der Spitzenabschnitt (102) aus einem Elastomermaterial gebildet sind.

## Revendications

1. Système de gaine d'introduction comprenant :
un élément de gaine (8) définissant une structure tubulaire (34) s'étendant entre une extrémité proximale (8a) et une extrémité distale (8b) de la gaine ; et
une partie de pointe (102) située à l'extrémité distale (8b) de l'élément de gaine (8), la partie de pointe (102) définissant une structure tubulaire s'étendant entre une extrémité proximale (102a) et une extrémité distale (102b) de la partie de pointe (102) et
ayant une lumière centrale (102c) s'étendant à travers elle, la partie de pointe (102) comprenant une caractéristique de came (104) formée comme une protubérance de la lumière centrale (102c) de la partie de pointe (102),
la caractéristique de came (104) comprenant un bord d'attaque (104a) et un bord de fuite adjacent (104b), le bord d'attaque (104a) étant adjacent à l'extrémité proximale (102a) de la partie de pointe (102) et le bord de fuite (104b) étant adjacent à l'extrémité distale (102b) de la partie de pointe (102) ;
la partie de pointe (102) pouvant être déplacée entre une configuration non déployée lorsque la caractéristique de came (104) n'est pas en prise et une configuration déployée lorsque la caractéristique de came (104) est en prise,
lorsque la partie de pointe (102) est dans la configuration non déployée, un diamètre de l'extrémité distale (102b) de la partie de pointe (102) étant inférieur à un diamètre de l'extrémité proximale (102a) de la partie de pointe (102) ; et
lorsque la partie de pointe (102) est dans la configuration déployée, au moins une partie de la caractéristique de came (104) définissant un diamètre interne (d₆) inférieur au diamètre (d₅) de la lumière centrale (38) de la gaine (8).

2. Système de gaine d'introduction selon la revendication 1, lorsque la partie de pointe (102) est dans la configuration non déployée, au moins une partie d'une surface extérieure de la partie de pointe (102) définissant une surface conique décroissante s'étendant entre les extrémités proximale et distale (102a, 102b) de la partie de pointe (102).

3. Gaine d'introduction selon l'une quelconque des revendications 1 et 2, l'élément de gaine (8) et la partie de pointe (102) formant une lumière centrale continue s'étendant entre l'extrémité proximale (8a) de l'élément de gaine (8) et l'extrémité distale (102b) de la partie de pointe (102).

4. Système de gaine d'introduction selon l'une quelconque des revendications 1 à 3, la caractéristique de came (104) étant formée comme une surface curviligne faisant saillie à partir de la lumière centrale (102c) de la partie de pointe (102) ;
la surface curviligne comprenant de préférence :
a) une surface convexe uniforme ; ou
b) une surface convexe asymétrique.

5. Système de gaine d'introduction selon l'une quelconque des revendications 1 à 4, une pente du bord d'attaque (104a) étant inférieure à une pente du bord de fuite (104b).

6. Système de gaine d'introduction selon la revendication 5, le bord d'attaque (104a) ayant un rayon de courbure qui est :
a) identique au rayon de courbure du bord de fuite (104b) ; ou
b) différent du rayon de courbure du bord de fuite (104b).

7. Système de gaine d'introduction selon l'une quelconque des revendications 1 à 6, la caractéristique de came (104) s'étendant de manière circonférentielle autour d'une surface intérieure de la partie de pointe (102),
la forme de la caractéristique de came (104) étant de préférence :
a) uniforme de manière circonférentielle autour de la surface intérieure de la partie de pointe (102) ; ou
b) non uniforme de manière circonférentielle autour de la surface intérieure de la partie de pointe (102).

8. Système de gaine d'introduction selon l'une quelconque des revendications 1 à 7, un diamètre le plus interne de la partie de pointe (102) étant défini par la caractéristique de came (104),
un diamètre le plus interne du système de gaine étant de préférence défini par la caractéristique de came (104).

9. Système de gaine d'introduction selon l'une quelconque des revendications 5 à 8, le bord de fuite (104b) étant en pente pour accepter un implant (12) ou une autre charge utile lorsqu'il est avancé à travers l'extrémité distale (102b) de la partie de pointe (102) vers l'extrémité proximale (102a) de la partie de pointe (102).

10. Système de gaine d'introduction selon l'une quelconque des revendications 1 à 9,
a) la caractéristique de came (104) ayant une surface lisse et continue inclinée pour faciliter le mouvement d'un implant (12) ou d'une autre charge utile à travers elle ; et/ou
b) une épaisseur de la partie de pointe (102) le long de la caractéristique de came (104) étant supérieure à une épaisseur le long d'une partie de pointe (102) excluant la caractéristique de came (104).

11. Système de gaine d'introduction selon l'une quelconque des revendications 1 à 10, au moins une partie de la partie de pointe (102) se déformant lors de la transition entre les configurations non déployée et déployée, permettant à l'extrémité distale (102b) de la partie de pointe (102) de s'évaser pour atteindre un plus grand diamètre dans la configuration déployée ;
une épaisseur de la caractéristique de came (104) restant de préférence constante dans la configuration déployée et non déployée.

12. Système de gaine d'introduction selon l'une quelconque des revendications 1 à 11,
a) la partie de pointe (102) étant sollicitée vers la configuration non déployée ; et/ou
b) lorsque la partie de pointe (102) est dans la configuration déployée, une partie d'une surface extérieure de la partie de pointe (102) formant une surface conique croissante.

13. Système de gaine d'introduction selon l'une quelconque des revendications 1 à 12, comprenant en outre un cathéter (14), le cathéter (14) étant au moins partiellement disposé à l'intérieur d'une lumière centrale (38) de l'élément de gaine (8) et pouvant être déplacé longitudinalement à travers celle-ci,
le cathéter (14) pouvant être déplacé à travers la lumière centrale (102c) de la partie de pointe (102),
la mise en prise entre le cathéter (14) et la caractéristique de came (104) faisant passer la partie de pointe (102) de la configuration non déployée à la configuration déployée.

14. Système de gaine d'introduction selon l'une quelconque des revendications 1 à 13, l'élément de gaine (8) pouvant être radialement déployé.

15. Système de gaine d'introduction selon l'une quelconque des revendications 1 à 14,
a) l'extrémité proximale (102a) de la partie de pointe (102) étant formée d'un matériau élastomère ; et/ou
b) l'élément de gaine (8) et la partie de pointe (102) étant formés d'un matériau élastomère.
